# EUROPÄISCHE PATENTSCHRIFT

(11) **EP 0 807 089 B1**
(45) Veröffentlichungstag und Bekanntmachung des Hinweises auf die Patenterteilung: **17.03.1999**
(21) Anmeldenummer: 96900871.3
(22) Anmeldetag: 30.01.1996
(51) Int. Cl.: C02F 11/12

(54) **Eindickzentrifuge zum Eindicken von Überschussschlamm**
Centrifuge for thickening waste water sludge
Machine centrifuge pour épaissir les boues des eaux usées

(30) Priorität: 30.01.1995 DE 19502856; 28.07.1995 DE 19527784
(43) Veröffentlichungstag der Anmeldung: 19.11.1997
(73) Patentinhaber: Vit, Robert, 95615 Marktredwitz (DE); Dohanyos, Michal, Prof., 163 00 Praha 6 (CZ); Zabranska, Jana, Dr., 160 00 Praha 6 (CZ); Kutil, Josef, Dipl.-Ing., 250 01 Brandys Nad Labem-Stara Boleslav (CZ)
(72) Erfinder: VIT, Robert, D-95615 Marktredwitz (DE); DOHANYOS, Michal, 163 00 Praha 6 (CZ); ZABRANSKA, Jana, 160 00 Praha 6 (CZ); KUTIL, Josef, 250 01 Brandys Nad Labem-Stara Boleslav (CZ)
(74) Vertreter: Winter, Brandl, Fürniss, Hübner, Röss, Kaiser, Polte, Kindermann Partnerschaft
(86) Internationale Anmeldenummer: DE9600130
(87) Internationale Veröffentlichungsnummer: WO9623736

(56) Entgegenhaltungen:
- EP-A- 0 537 595
- DE-A- 4 030 668
- DE-A- 4 205 739
- US-A- 4 342 650
- US-A- 5 380 445
- PATENT ABSTRACTS OF JAPAN vol. 017, no. 171 (C-1044), 2.April 1993 & JP,A,04 331000 (KUBOTA CORP), 18.November 1992,
- PATENT ABSTRACTS OF JAPAN vol. 018, no. 191 (C-1186), 4.April 1994 & JP,A,05 345192 (INA SHOKUHIN KOGYO KK), 27.Dezember 1993,

## Beschreibung

Die vorliegende Erfindung betrifft eine Eindickzentrifuge zum Eindicken von Überschußschlamm gemäß Patentanspruch 1, sowie ein Verfahren zur Verminderung der Faulschlammenge bzw. Restschlammenge in Kläranlagen gemäß Anspruch 43.

Die erfindungsgemäßen Vorrichtungen sowie das erfindungsgemäße Verfahren dienen dazu, die Schlammproduktionsmasse in Kläranlagen zu vermindern, die Entwässerung zu verbessern und die Biogasfermentation bei einer anaeroben Methanfermentation des Schlammes, der Abfallbiomasse sowie bei der anaeroben Reinigung von Abfallwasser zu beschleunigen.

Bei der biologischen Abfallreinigung entstehen relativ große Schlammengen, wobei der Umgang mit ihnen und deren wirtschaftliche Nutzung oft zu Problemen führt. Bei der aeroben biologischen Reinigung des Abwassers entstehen wenigstens zwei Schlammarten: Primärschlamm - jener Schlamm, der bei der primären Ablagerung des zugeleiteten Abwassers entsteht und einer raschen Zersetzung unterliegt - und als zweiter Schlammtyp der belebte Überschußschlamm, welcher nach der biologischen Reinigungsstufe des Abwassers anfällt. Dabei handelt es sich vorwiegend um ein Gemisch von Mikroorganismen, die bei der Abwasserreinigung entstehen und deren Anzahl von der Menge der beseitigten Verunreinigungen sowie den Verfahrensgegebenheiten bei der Reinigung abhängig ist. Beide Schlammarten werden in den meisten Kläranlagen, vor allem in den großen, durch eine anaerobe Methanfermentation verarbeitet.

Die anaerobe Methanfermentation organischer Stoffe ist ein Prozeß, bei dem eine Mischkultur von Mikroorganismen unter anaeroben Bedingungen stufenweise eine biologisch abbaufähige organische Masse zersetzt. Die Endprodukte dieser Zersetzung sind Methan, Kohlendioxid, Schwefelwasserstoff (Sulfan), Stickstoff, Wasserstoff, die entstandene Biomasse und ein stabilisierter organischer Stoff (ein weiter nicht mehr zersetzbarer Restbestand).

Zu den wichtigsten Faktoren, die den Ablauf der anaeroben Zersetzung beeinflussen, gehören die Zusammensetzung des Substrates (von ihm abhängig die spezifische Produktion sowie die Zusammensetzung des Biogases), die Anwesenheit von Nährstoffen, der pH-Wert, die Pufferkapazität sowie die Temperatur. Die Wirtschaftlichkeit dieses Prozesses ist von der Trockensubstanzkonzentration (Feststoffkonzentration) des zu bearbeitenden Materials abhängig. Aus diesem Grund ist das eingehende Material oft einem Verdickungsprozeß unterworfen, wobei dies entweder maschinell (Zentrifugen, Pressen u.ä.) oder mit der Hilfe von Schwerkraft geschieht.

Für einen günstigen Verlauf der anaeroben Zersetzung ist die Anwesenheit einer Reihe von anorganischen Nährstoffen von Bedeutung. Wichtig ist auch die Anwesenheit einer Reihe von Wachstumsfaktoren (Vermehrungsfaktoren) sowie von Vitaminen und Enzymen. Für die anaerobe Zersetzung sind vor allem hydrolytische Enzyme von Bedeutung, welche eine Vielzahl von Stoffen, wie z.B. auch feste und hochmolekulare organische Stoffe, abbauen können. Einige dieser Stoffe können die Mikroorganismen selbst synthetisieren, andere müssen von außen zugesetzt werden.

Eine Labormöglichkeit, die Biogasentwicklung bei der anaeroben Methanfermentation von Schlamm, Abwasser sowie sonstigen organischen Stoffen zu stimulieren, beschreibt die CZ-PS 242 979. Das in diesem Patent offenbarte Verfahren besteht darin, daß dem zur Fermentation bestimmten Material, bzw. direkt in den anaeroben Reaktor, als Stimulierungsmittel eine getrennt von der Labor-Abwasseraufbereitungsanlage mechanisch oder physikalisch behandelte Mikroorganismen-haltige Biomassensuspension von zwischen 0,1 bis zu 10 Gew.-%, vorzugsweise 5 Gew.-%, bezogen auf die organischen Trockensubstanz des zu bearbeitenden Materials, zugefügt wird.

Bei der lytischen Behandlung (mechanische und physikalische Zerstörung von Zellen) der Biomassensuspension gemäß dem Stand der Technik wird teilweise der Inhalt der Zellen dieser Mikroorganismen freigesetzt.

Der Inhalt von Mikroorganismenzellen oder Zellen anderer Organismen, der in Folge der Zerstörung von Zellwänden und/oder Zellmembranen in die Lösung frei wird (auch Zellysat genannt), wirkt sich förderlich bzw. stimulierend auf den Prozeß der biologischen Zersetzung der organischen Stoffe aus. Die Lyse der Zellen verläuft einerseits auf natürliche Weise (Autolyse) bei abgestorbenen Zellen, andererseits mit Hilfe von hydrolytischen Enzymen, welche durch Fermentationsbakterien in die Lösung freigesetzt werden, und ferner durch die künstliche Zerlegung von Mikroorganismen bzw. Organismen mit Hilfe von physikalisch-chemischen oder mechanischen Methoden.

Das Zellysat stimuliert einerseits die Funktion und das Wachstum von Mikroorganismen, andererseits enthält es selbst eine Reihe von Enzymen, die direkt zum Abbau organischer Stoffe erforderlich sind. Das Lysat stimuliert die Tätigkeit einiger Bakterien, die Wasserstoff und Kohlendioxid, Essigsäure, Propionsäure, usw. umsetzen und/oder freisetzen können. Außerdem verstärkt es die Zersetzung organischer Masse und erhöht die Biogasproduktion bei anaeroben Methanisierungsprozessen, was eine Verringerung der Gesamtmenge des produzierten Schlammes sowie die Erhöhung der Biogasproduktion zur Folge hat. Das Zellysat kann ebenfalls die Produktion von überschüssigem belebtem Schlamm bei der Anwendung im Belebungsbecken bedeutend beeinflussen.

Die DE-A1-40 30 668 beschreibt eine Vorrichtung zum Eindicken von Abwasserschlamm, die einen Desintegrator umfaßt, in dem der Schlamm durch Mahlvorgänge desintegriert wird. In diesem Desintegrator werden die Zellen durch mechanische Zellwand-Zerstörung lysiert.

Die Aufbereitung von Zellysaten im Labormaßstab gemäß dem Stand der Technik ist sowohl bezogen auf die dafür benötigte Anlage als auch die dafür notwendige Energiemenge aufwendig. Dies trifft für sämtliche bisher bekannte Methoden der Zellzerlegung bzw. Destruktion zu, wie z.B. dies bei der Desintegrierung mittels mechanischer Methoden (Mahlen, Zerreiben, Pressen), Sonifizierung (Ultraschallbehandlung), Kavitation, wiederholtes Einfrieren und Auftauen, Wärmeeinwirkung u.ä. der Fall ist.

Das oben beschriebene Verfahren nach dem Stand der Technik der CZ-PS 242 979 hat vor allem den Nachteil, daß es eine separate Zubereitung des Zellysates - außerhalb der eigentlichen Klärvorrichtung - nötig macht. Wie bereits oben erwähnt ist dieses Verfahren nur für einen Labormaßstab geeignet; eine Übertragung auf ein großtechnisches Verfahren ist aufgrund der großen Platzanforderungen, der hohen Energie- und Investitionskosten und dem zusätzlichen Personalaufwand nicht praktikabel. So ist es beispielsweise weder wirtschaftlich noch technisch sinnvoll Schlammengen, die im Tonnenmaßstab anfallen, zyklisch einzufrieren und wieder aufzutauen.

Ausgehend von dem Stand der Technik der CZ-PS 242 979 ist es somit Aufgabe der vorliegenden Erfindung, die in Kläranlagen anfallenden Schlammengen unter großtechnischen Bedingungen auf eine weniger aufwendige und auf eine kostengünstigere Weise signifikant zu verringern.

Die Lösung dieser Aufgabe erfolgt vorrichtungstechnisch durch die kennzeichnenden Merkmale des Patentanspruches 1 und verfahrenstechnisch durch die kennzeichnenden Merkmale des Patentanspruchs 43.

Die vorliegende Erfindung stellt eine Eindickzentrifuge zum Eindicken von Überschußschlamm zur Verfügung. Sie ist baulich so gestaltet, daß die Zellen der in dem Klärschlamm enthaltenen Organismen, insbesondere Mikroorganismen, bei der Eindickung wenigstens teilweise lysiert werden.

Durch die erfindungsgemäße Vorrichtung wird die Schlammproduktion in Kläranlagen signifikant verringert. Dadurch, daß die Zellen der Mikroorganismen in der Eindickzentrifuge lysiert werden, wird es außerdem möglich, diese Vorrichtungen auch im großtechnischen Bereich einzusetzen.

Dies geschieht verfahrenstechnisch, wie in der deutschen Patentanmeldung 195 02 856.2 desselben Anmelders mit dem Titel "Vorrichtung und Verfahren zur Verminderung der Schlammproduktion in Kläranlagen" vom 30. Januar 1995 beschrieben, auf welche hiermit diesbezüglich vollinhaltlich Bezug genommen wird. Es wird ebenfalls auf die deutsche Patentanmeldung 195 27 784.8 desselben Anmelders mit dem Titel "Vorrichtung zum Eindicken und Fördern von Abwässerschlämmen" vom 28. Juli 1995 verwiesen, auf welche ebenfalls hiermit vollinhaltlich Bezug genommen wird.

Dabei besteht ein besonderer Vorteil darin, daß die Eindickzentrifuge gleichzeitig zum Eindicken wie auch zum Lysieren der Zellen verwendet werden kann, was die Vorrichtung bzw. das Verfahren ökonomischer macht. Darüber hinaus ist in der erfindungsgemäßen Vorrichtung ein kontinuierliches Lysieren der Zellen bei einem relativ geringen Schlammdurchsatz möglich, wodurch vermieden wird, daß große Schlammengen gleichzeitig aufbereitet werden müssen.

Schließlich stellt die vorliegende Erfindung ein Verfahren zur Verminderung der Faulschlammenge bzw. Restschlammenge in Kläranlagen zur Verfügung, welches die Schritte gemäß Anspruch 43 umfaßt.

Durch die erfindungsgemäße Vorrichtung bzw. das erfindungsgemäße Verfahren wird die Schlammproduktion in Kläranlagen signifikant verringert. Dadurch daß die Zellen der Mikroorganismen in der Klärvorrichtung selbst bzw. in der Eindickzentrifuge lysiert werden, wird es außerdem möglich, diese Vorrichtung bzw. dieses Verfahren auch im großtechnischen Bereich einzusetzen. Dabei besteht ein Vorteil darin, daß die Eindickzentrifugen gleichzeitig zum Eindicken des Schlammes wie auch zum Lysieren der Zellen verwendet werden können, was die Vorrichtungen bzw. das Verfahren ökonomischer macht. Darüber hinaus ist in der Vorrichtung bzw. dem Verfahren ein kontinuierliches Lysieren der Zellen bei einem relativ geringen Schlammdurchsatz möglich, wodurch vermieden wird, daß große Schlammengen gleichzeitig aufbereitet werden müssen.

Da bei der erfindungsgemäßen Vorrichtung bzw. dem erfindungsgemäßen Verfahren keine aufwendige Aufbereitung des Zellysates außerhalb der Klärvorrichtung mehr nötig ist, fallen somit auch keine erhöhten Kosten und kein erhöhter Energie- und Personalaufwand an. Außerdem wird die Leistung des anaeroben Reaktors erhöht und der Abbau organischer Stoffe beschleunigt, Problemstoffe, wie z.B. verschiedene Xenobiotika oder Giftstoffe, werden verstärkt abgebaut, die Produktion an brennbarem Gas wird erhöht und die Energiebilanz wird im Vergleich zum Stand der Technik ebenfalls verbessert.

Die oben erwähnten Nachteile des Standes der Technik werden somit durch die erfindungsgemäße Eindickzentrifuge bei der Behandlung von Schlamm und Abfallbiomasse durch anaerobe Methanfermentation sowie bei der anaeroben Abwasserreinigung wenigstens weitgehend beseitigt.

Die vorliegende Erfindung umfaßt eine Eindickzentrifuge zum Eindicken von Abwässerschlämmen, wobei an der Zentrifuge wenigstens eine Lysiereinrichtung zum Aufbrechen der Zellen von in den Abwässerschlämmen enthaltenen Organismen vorgesehen ist.

Bei der bevorzugten Ausführungsform der vorliegenden Erfindung ist die Lysiereinrichtung ein integraler Bestandteil der Zentrifuge und ist vorzugsweise am Zentrifugenausgang vorgesehen.

Selbstverständlich kann jedoch auch eine separate Lysiereinrichtung sowohl am Zentrifugeneingang als auch am Zentrifugenausgang oder in Leitungssystemen unabhängig von einer direkten baulichen Verknüpfung mit der Zentrifuge vorgesehen sein.

Der besondere Vorteil der vorliegenden Erfindung liegt darin begründet, daß die in der Abwassertechnik ohnehin verwendeten Eindickungsvorrichtungen, insbesondere Zentrifugen, nur eine relativ kleine bauliche Änderung erfahren müssen, damit sie die Voraussetzungen der vorliegenden Erfindung erfüllen.

Eine Vorrichtung gemäß Anspruch 3 zeichnet sich im wesentlichen dadurch aus, daß sie einen hohen Förder- und Eindickungswirkungsgrad hat, wenn sie eine rotierende Förderschnecke sowie einen rotierenden Mantel aufweist, wobei der rotierende Mantel lediglich eine noch bessere Förderung des Schlammes ergibt und eine Zusetzung oder Verbackung des Schneckenförderers mit dem Mantel der Zentrifuge weitgehend verhindert.

Dieser Effekt wird noch dadurch verstärkt, daß die Schneckenfördereinrichtung und der Mantel der Zentrifuge mit unterschiedlichen Umfangsgeschwindigkeiten gemäß Anspruch 4 rotieren.

Dabei liegt die Umfangsgeschwindigkeit des Schneckenförderers um ca. 10 UPM höher als die Umfangsgeschwindigkeit des Zentrifugenmantels.

Die Lysiereinrichtung kann wie bereits oben erwähnt am Zentrifugeneingang und/oder Zentrifugenausgang und/oder in der Zentrifuge integriert vorgesehen sein. Dies hat den Vorteil, daß hierdurch eine Vielzahl von Maßnahmen zur Verfügung steht, die vorliegende Erfindung an die jeweiligen Verhältnisse und Konsistenzen und Zusammensetzungen der Abwässerschlämme in den unterschiedlichsten Kläranlagen anzupassen.

Um größere Fremdkörper von den naturgemäß relativ engen Durchtrittsräumen innerhalb der Lysiereinrichtungen fernzuhalten, ist es häufig sinnvoll eine Siebeinrichtung in der Schlammzuführung für die Lysiereinrichtung vorzusehen. Durch diese Maßnahme wird weitgehend eine Beschädigung der Lysiereinrichtung durch etwaige größere Teile wie Steine und nicht verrottbare Abfallmaterialien vermieden.

Derartige Siebeinrichtungen sind vorzugsweise derart angeordnet, daß sie leicht während des Betriebes der Kläranlage und der Zentrifuge ausgetauscht werden können. So ist etwa die Möglichkeit einer zweifachen Schlammzuführung nach Art eines Bypasses vorgesehen, wobei der Schlammweg über den Bypass mit intakter Siebeinrichtung geführt wird, wenn die Siebeinrichtung der anderen Leitung gereinigt werden muß. Die Lysiereinrichtung selbst kann mehrere Bauarten umfassen. Welche spezielle Lysiereinrichtung gewählt werden wird, hängt von den jeweiligen Schlammverhältnissen ab, so daß hier eine breite Palette zur Verfügung steht, welche Zentrifugen mit welchen Lysiereinrichtungen für welchen Zweck ausgestattet werden.

Derzeit liegen Erfahrungen mit sieben unterschiedlichen Lysiereinrichtungen vor.

So ist gemäß Anspruch 5 die Lysiereinrichtung beispielsweise als Friktionsmahlwerk ausgebildet, was bedeutet, daß im wesentlichen Scher- und Reibekräfte nach Art eines Zermahlens mittels eines Mühlsteins für die lysierende Wirkung verantwortlich sind.

Ein solches Friktionsmahlwerk als Lysiereinrichtung ist beispielsweise in Anspruch 6 an der erfindungsgemäßen Vorrichtung vorgesehen, wobei dieses Friktionsmahlwerk wenigstens eine Mahlscheibe aufweist, welche zum Zerreiben des Schlammes und der darin enthaltenen Zellen dient.

In einer solchen Vorrichtung wird der durch die Zentrifuge entwässerte Schlamm vorzugsweise zwischen zwei aufgerauhten Oberflächen mechanisch zerrieben, wobei durch die hohen Scherkräfte die innerhalb des Schlammes und der Zellen zwischen den reibenden Oberflächen entstehen, die Zellen der Mikroorganismen und höherer Organismen zerrissen und damit lysiert werden.

Vorzugsweise ist ein solches Friktionsmahlwerk als Lysiereinrichtung gemäß Anspruch 7 ausgebildet, wobei eine Mahl scheibe mit dem rotierenden Mantel der Zentrifuge verbunden ist und somit mit diesem rotiert und sich daher gegen eine weitere feststehende Mahlscheibe bewegt. Dabei liegt der Abstand beider Mahlscheiben etwa im Bereich von 0,5-5 mm.

Eine derartige Konstruktion einer Lysiereinrichtung hat den Vorteil, daß er mit wenig Dichtaufwand auskommt, daß der Abstand zwischen den beiden Mahlscheiben einstellbar ist, wodurch der Lysiergrad einstellbar ist und daß eine derartige Lysiereinrichtung auch mehrstufig aufbaubar ist, wobei unterschiedliche Oberflächen der Mahlscheiben beziehungsweise unterschiedliche Abstände zwischen den Mahlscheiben der einzelnen Stufen verwendet werden können.

Aufgrund des relativ geringen Abstands beider Mahlscheiben, ist es sinnvoll, größere Fremdkörper innerhalb des Schlammes durch ein Sieb im Zulauf zurückzuhalten, um Beschädigungen der Lysiereinrichtung zu vermeiden.

Eine Erhöhung der Scherkräfte, die an den im Abwasser enthaltenen Zellen entstehen, findet insbesondere dadurch statt, daß die Mahlscheiben an ihren Mahl flächen Vertiefungen, insbesondere Nuten aufweisen, wobei diese Nuten vorzugsweise einen Anstellwinkel gegenüber der Radialrichtung aufweisen. Dabei ist es besonders bevorzugt, daß diese Vertiefungen in der rotierenden Mahlscheibe auftreten, weil hierdurch eine gewisse pumpende Wirkung sowie eine Ausbildung von relativ großen Druckgradienten und damit eine erhöhte Scherung und hierdurch bedingt eine erhöhte Lysierwirkung auftritt.

Wenn Anstellwinkel der Vertiefungen beziehungsweise Nuten gegenüber der Radialrichtung in der Oberfläche der Mahlscheiben, vorzugsweise der rotierenden Mahlscheibe, vorgesehen sind, kann diese Pumpwirkung je nach Winkel verstärkt oder abgeschwächt werden.

Eine Anordnung von nebeneinander liegenden Nutenreihen auf Lücke gemäß Anspruch 10 hat den Vorteil, daß die Scherwirkung und die unmittelbare Lysierwirkung auch durch Erzeugung von Turbulenzen nochmals verstärkt wird.

Die Verweilzeit des Schlammes in einem Typ dieser Lysiereinrichtung kann noch dadurch gesteuert werden, daß gemäß Anspruch 11 ein Wehr am Schlammauslaß vorgesehen ist, dessen Höhe das Lysiervolumen von Schlamm in der Lysiereinrichtung und damit die Verweilzeit steuert.

Hierdurch kann wiederum die Lyseeffizienz bedarfsgerecht gesteuert verändert bzw. eingestellt werden.

Ein weiterer Typ einer erfindungsgemäßen Vorrichtung weist eine Lysiereinrichtung auf, die ebenfalls als Friktionsmahlwerk ausgebildet ist, jedoch anstelle einer Mahl scheibe einen Mahlkonus gemäß Anspruch 12 zum Zerreiben des Schlammes und der darin enthaltenen Zellen aufweist.

Die lysierende Wirkung ergibt sich hier ähnlich wie bei der zuvor beschriebenen Ausführungsform der erfindungsgemäßen Vorrichtung, nämlich am Ausgang von Zentrifuge tritt ebenfalls Schlamm in den Mahlkonus ein und wird zwischen einem rotierenden Außenkonus, der wenigstens eine Mahlfläche aufweist und einem vorzugsweise feststehenden Innenkonus zerrieben. Es ist auch eine kinematische Umkehr möglich.

Gemäß den Ansprüchen 14, 15 und 16 können sowohl der rotierende Außenkonus als auch der feststehende Innenkonus bei Bedarf Oberflächenvertiefungen, insbesondere Nuten aufweisen, welche hier vorzugsweise in Richtung der Mantellinie des Mahlkonus oder in einem Anstellwinkel hierzu angeordnet sind, wobei diese Vertiefungen dieselben Lyse-verstärkenden Wirkungen haben, wie die Vertiefungen auf der Mahlscheibe in der oben beschriebenen Ausführungsform.

Besonders bevorzugt ist bei der Ausführungsform der vorliegenden Erfindung, welche einen Mahlkonus als Lysiereinrichtung verwendet, daß der Spalt zwischen rotierendem Außenkonus und dem feststehenden Innenkonus gemäß Anspruch 17 über einen Anschlag und Federn unter Umständen während des Betriebs veränderlich und einstellbar ist.

Die Ausführungsform der erfindungsgemäßen Vorrichtung, welche einen Mahlkonus als Lysiereinrichtung verwendet, hat den Vorteil, daß sie einfach nachzustellen ist und daß sie größeren Fremdkörpern aufgrund der gefederten Lagerung des Innenkonus ausweichen kann.

Gemäß Anspruch 18 weist die erfindungsgemäße Vorrichtung zum Eindicken von Abwasserschlämmen als Lysiereinrichtung eine sogenannte Profilreibe auf. Bei dieser Profilreibe ist gemäß Anspruch 19 eine feststehende Außenhülle und eine rotierende Reibfläche vorgesehen, welche mit dem rotierenden Mantel der Zentrifuge verbunden ist, wobei ein enger Spalt zwischen der feststehenden Außenhülle und der Reibfläche existiert.

Schlamm, welcher durch eine Austrittsöffnung durch die rotierende Reibfläche hindurch in den Spalt zwischen Außenhülle und Reibfläche tritt, wird gezwungen durch diesen engen Abstand zu fließen, wobei der große Druck bewirkt, daß so hohe Scherkräfte entstehen, daß die Zellwände von Bakterien und anderen Mikroorganismen wie beispielsweise Protozoen platzen und somit lysiert werden.

Gemäß Anspruch 20 kann die rotierende Reibfläche auf ihrer Oberfläche Vertiefungen insbesondere wellenförmige Vertiefungen aufweisen, welche wiederum zu Erhöhung der Scherkräfte dienen.

Gemäß Anspruch 21 kann die Reibfläche auf beiden Seiten ihrer Austrittsöffnung unterschiedliche Neigungen in Richtung auf die Außenhülle aufweisen, wobei derjenige Teil der Reibfläche, welcher zum geschlossenen Ende der Vorrichtung zeigt enger ist als derjenige Teil der Reibfläche, der zur Schlammauslaßöffnung zeigt, wodurch ein Druckgradient in Richtung Schlammauslaß entsteht.

Durch diesen Druckgradienten wird einerseits eine Förderwirkung in Richtung des Schlammauslasses erzielt und andererseits die auf die Mikroorganismen wirkenden Scherkräfte nochmals erhöht, wodurch die Lysierwirkung insgesamt verstärkt wird.

Gemäß Anspruch 22 ist die Schlammzuführung zur Profilreibe vorzugsweise zentrisch vorgesehen und gemäß Anspruch 23 beträgt der Abstand zwischen Reibfläche und Außenhülle etwa minimal 2 mm bis maximal 10 mm.

Die Lysiereinrichtung als Profilreibe auszubilden hat insgesamt die Vorteile, daß praktisch keine Dichtigkeitsprobleme auftreten, daß sie einfach zu bauen ist, daß keine Metallreibung auftritt und daß größere Teilchen seitlich ausweichen können, so daß unter Umständen eine Siebeinrichtung entfallen kann.

Darüber hinaus kann konstruktionstechnisch bedingt eine niedrige Bauhöhe eingehalten werden, so daß ein relativ platzsparender Aufbau ermöglicht wird.

Der Mechanismus der Lyse ergibt sich vorwiegend durch Quetschen und Zerreiben und die dadurch entstehenden Scherkräfte.

Gemäß Anspruch 24 ist eine weitere Ausführungsform der vorliegenden Vorrichtung zum Eindicken von Abwasserschlämmen beansprucht, nämlich eine solche, bei welcher die Lysiereinrichtung als Walzenquetsche ausgebildet ist.

Bei dieser Ausführungsform der erfindungsgemäßen Vorrichtung werden Walzen nach Art eines Wälzlagers von einem rotierenden Teil auf der Innenwand einer feststehenden Außenhülle gemäß Anspruch 25 in Innenumfangsrichtung der Außenhülle gerollt. Hierbei liegt praktisch ein feststehender Außenzylinder vor, welcher Ausnehmungen aufweist, in welchen sich die Walzen bewegen. Darüberhinaus weist das rotierende Teil, welches mit dem Mantel der Zentrifuge verbunden ist, ebenfalls Ausnehmungen auf, in welchem ein anderer Walzensatz gelagert ist und über die Innenoberfläche in Innenumfangsrichtung der Außenhülle gerollt wird.

Wenn Schlamm aus dem Zentrifugenausgang in eine solche Lysiereinrichtung tritt, gerät er in die Zwischenräume der Walzen und wird von diesen mitgenommen, überrollt und hierbei gequetscht, wobei wiederum hohe Scherkräfte auftreten, die ausreichen, um die Zellen von in den Abwässerschlämmen enthaltenen Organismen, insbesondere Mikroorganismen, besonders fein zu lysieren.

Gemäß Anspruch 26 weist ein Walzensatz wenigstens zehn Walzen auf, wobei insgesamt wenigstens zwei Walzensätze bevorzugt sind.

Eine erfindungsgemäße Vorrichtung mit einer Walzenquetsche als Lysiereinrichtung weist eine gute Zerkleinerung und Lysewirkung auf, wobei die Bauhöhe relativ gering gehalten werden kann.

Für eine derartige Lysiereinrichtung sind jedoch eine relativ große Genauigkeit bei der Konstruktion der einzelnen Teile sowie eine relative hohe Laufruhe erforderlich, da bereits kleinere nicht zerquetschbare mineralische Teile die Vorrichtung fressen lassen, so daß bei dieser Lysiereinrichtung, vorzugsweise ein Sieb vor der Zentrifuge angeordnet werden sollte.

Anspruch 27 beansprucht eine Vorrichtung zum Eindicken von Abwässerschlämmen bei welcher die Lysiereinrichtung als sogenannte Passiertrommel ausgebildet ist.

Gemäß Anspruch 28 weist eine derartige Passiertrommel mehrere Passierelemente auf, welche in einer feststehenden Außenhülle mit einem vorzugsweise einstellbaren Abstand zur Innenwand der Außenhülle rotieren. Gelangt Schlamm aus dem Zentrifugeneingang zwischen die einzelnen Passierelemente, so wird er durch den engen Spalt zwischen Außenhülle und den einzelnen Passierelementen, welche beispielsweise als Passierscheiben ausgebildet sein können, gequetscht und durch die hierdurch entstehende hohe Scherbeanspruchung werden die Mikroorganismenzellen zerstört.

Die Vorteile einer derartigen Lysiereinrichtung liegen darin begründet, daß ein sehr kleiner Bauraum möglich ist und daß mehrere derartiger Lysiereinrichtungen hintereinander geschaltet werden können, um die Lysierwirkung, vorzugsweise stufenweise, zu erhöhen.

Da bei einer derartigen Konstruktion der Lysiereinrichtung jedoch größere Fremdkörper zu Beschädigungen der Passierelemente führen können, ist es sinnvoll größere Fremdkörper vor der Zentrifuge auszusieben.

Gemäß Anspruch 29 ist eine Vorrichtung zum Eindicken von Abwasserschlämmen beansprucht, welche als Lysiereinrichtung ein Schneidwerk aufweist.

Ein solches Schneidwerk weist gemäß Anspruch 30 rotierende Schneidelementereihen, insbesondere Messerreihen, feststehende Schneidelemente, insbesondere Messerreihen sowie ein Wehr am Schlammauslaß der Lysiereinrichtung auf und die Schneidelemente sind derart angeordnet, daß sie ineinandergreifen, ohne sich zu berühren.

In der Regel tritt Schlamm am Zentrifugenausgang in das Innere des Schneidwerks ein, wobei der Schlamm die Zentrifuge mit etwa 50 m/s verläßt und zunächst auf eine abgeschrägte Oberfläche auftrifft, wodurch bereits ein Teil der Zellen durch den Auftreffimpuls zerstört wird. Der Schlamm gelangt dann über einen Kanal weiter in das Schneidwerk wo er durch eine Vielzahl von rotierenden Messern einerseits und feststehenden Messern andererseits senkrecht zu einer Rotationsebene hindurchgedrückt wird und der Schlamm durch die vorzugsweise als Messer ausgebildeten Schneidelemente enormen Zerteil- und Scherkräften ausgesetzt ist, wodurch die Zellen, welche in den Abwasserschlämmen enthalten sind, lysiert werden. Die hauptsächliche physikalische Wirkung liegt dabei im Zerschneiden und Zerschlagen der Zellen.

Gemäß Anspruch 31 kann der Abstand zwischen den rotierenden und den feststehenden Messerreihen einstellbar sein, wodurch die lysierende Wirkung des Schneidwerks an die Eigenschaften des Schlammes angepaßt werden kann.

In der Praxis hat sich herausgestellt, daß gemäß Anspruch 32 die Messer mit einer Geschwindigkeit von ca. 50-100 m/s, vorzugsweise ca. 80 m/s rotieren.

Diese Geschwindigkeitswerte sorgen einerseits für eine ausreichende lysierende Wirkung und andererseits für eine relativ hohe Standzeit der Schneidelemente.

Gemäß Anspruch 33 sind die Messerreihen vorzugsweise auf Lücke angeordnet, wobei nebeneinanderliegende Messerreihen so angeordnet sind, daß Bereiche, an denen eine Messerreihe unterbrochen ist, die benachbarten Messerreihen keine Unterbrechung aufweisen, wodurch die lysierende Wirkung deutlich verstärkt wird.

Eine weitere Möglichkeit zur Erhöhung der lysierenden Wirkung liegt darin, daß gemäß Anspruch 34 die Enden der Schneidelemente, insbesondere der Messerenden, einen Anstellwinkel zur Rotationsrichtung des Schneidwerks aufweisen, so daß hierdurch ein Teil des Schlammes der vorherigen Schneidelementereihe, insbesondere Messerreihe, wieder zugeführt wird, so daß sich quasi eine Pumpwirkung in Richtung auf die Zentrifuge ergibt und hierdurch die Verweilzeit des Schlammes innerhalb des Schneidwerkes bei Bedarf drastisch erhöht werden kann.

Die Verweilzeit ist ebenfalls über die Höhe des Wehrs am Schlammauslaß gemäß Anspruch 35 einstellbar.

Die Vorteile, die Lysiereinrichtung der erfindungsgemäßen Vorrichtung als Schneidwerk auszubilden liegen darin begründet, daß diese Art von Lysiereinrichtung zur Wirkungsgradverbesserung bezüglich der Lyse der Zellen von Mikroorganismen beliebig ausbaubar ist, so zum Beispiel durch Erhöhung der Anzahl der Messer oder auch durch Ändern der Anstellwinkel der Messerenden, so daß eine ausgezeichnete Zerkleinerung auftritt und die Verweilzeit über mehrere konstruktive Parameter wie beispielsweise Wehrhöhe, Anstellwinkel der Messerenden sowie Abstand zwischen rotierenden und feststehenden Schneidelementen, Länge der Schneidelemente in Umfangsrichtung und Höhe der Messer einstellbar ist.

Da die Messer als Schneidelemente relativ empfindlich sind, sollte vorzugsweise eine Aussiebung von größeren Fremdkörpern, vorzugsweise bereits im Zentrifugeneingang bzw im Eingang der Fördervorrichtung, erfolgen.

Um die Standzeiten der Schneidelemente zu erhöhen, sollten hier vorzugsweise harte Materialien zum Einsatz kommen, wie beispielsweise Chrom-Vanadin-Molybdän-Legierungen oder Titan sowie auch mit Wolframcarbid- oder Diamantsplitter besetzte Schneiden.

Gemäß Anspruch 36 weist eine erfindungsgemäße Vorrichtung ein Stiftmahlwerk als Lysiereinrichtung auf.

Grundsätzlich ist ein derartiges Stiftmahlwerk ähnlich aufgebaut wie ein Schneidwerk, jedoch mit der Maßgabe, daß keine Schneidelemente sondern Stifte auf einem rotierenden und einem feststehenden Teil angeordnet sind, wobei diese Stifte welche in Form von Stiftreihen angeordnet sind gemäß Anspruch 37 ineinander greifen, wobei die rotierenden Stiftreihen die feststehenden Stiftreihen nicht berühren.

Bei dieser Art der Lysiereinrichtung tritt am Zentrifugenausgang bzw. am Ausgang der Fördervorrichtung Schlamm in das Innere des Stiftmahlwerks ein, wobei der Schlamm durch die engen Spalte der ineinandergreifenden und teilweise rotierenden Stifte gezwungen wird, wodurch die im Schlamm enthaltenen Zellen durch Aufprall und Zerschlagen lysiert werden. Darüber hinaus treten zwischen den einzelnen Stiften hohe Scherkräfte auf, wodurch ebenfalls eine Lyse der Zellen einsetzt.

Gemäß Anspruch 38 ist der Abstand zwischen den feststehenden und rotierenden Stiftreihen einstellbar, wodurch der Lysierungsgrad eingestellt werden kann.

Gemäß Anspruch 39 rotieren die Stifte mit einer Geschwindigkeit von ca. 50-100 m/s, vorzugsweise ca. 80 m/s.

Gemäß Anspruch 40 sind nebeneinander liegende Stiftreihen auf Lücke angeordnet, so daß Bereiche an denen keine Stifte in einer Stiftreihe vorhanden sind, die benachbarten Stiftreihen Stifte aufweisen. Die Stifte können gemäß Anspruch 41 auch einen Winkel zur Drehachse einschließen. Hierdurch wird ebenfalls eine Erhöhung der lysierenden Wirkung geschaffen.

Gemäß Anspruch 42 ist die Höhe des Wehrs am Schlammauslaß dieser Art der Lysiereinrichtung einstellbar, wodurch wiederum die Verweilzeit des Schlammes innerhalb der Lysiereinrichtung an die Bedürfnisse angepaßt werden kann.

Eine erfindungsgemäße Vorrichtung zum Eindicken von Abwasserschlämmen mit einer Lysiereinrichtung, die als Stiftmahlwerk ausgebildet ist, hat den Vorteil, daß die Verweilzeit einstellbar ist, daß sie beliebig ausbaubar ist und die Wirkung beispielsweise durch die Zahl der Schlagstifte sehr leicht zu variieren ist, und daß Schlagstifte bei Bedarf leicht austauschbar sind.

Auch bei dieser Ausführungsform ist es sinnvoll Fremdkörper, vorzugsweise im Zentrifugen- bzw. Pumpenzulauf, über eine Siebeinrichtung zurückzuhalten.

Eine Kläranlage, (nicht Bestandteil der vorliegenden Erfindung) weist folgendes auf: wenigstens ein Absetzbecken mit wenigstens einem Zufluß sowie wenigstens eine Fördereinrichtung, wenigstens eine aerobe Belebungseinrichtung und wenigstens einen anaeroben Reaktor aufweist, wobei die Kläranlage wenigstens eine Vorrichtung zum Eindicken von Abwasserschlämmen aufweist, an welcher eine Lysiereinrichtung vorgesehen ist.

Als Lysiereinrichtung kann neben den bereits beschriebenen auch eine Unterdruckeinrichtung und/oder Überdruckeinrichtung, insbesondere Preßeinrichtung und/oder Zerreibeinrichtung und/oder Beschallungseinrichtung, vorzugsweise Ultraschalleinrichtung und/oder Vibrationseinrichtung verwendet werden. Durch diese verschiedenen Möglichkeiten kann die Anlage optimal an die Beschaffenheit des jeweiligen Abwassers und/oder Klärschlammes angepaßt werden. Weiterhin kann je nach Bedarf stärker oder weniger stark lysiert werden, womit die erfindungsgemäße Vorrichtung bzw. das damit durchgeführte Verfahren sehr anpassungsfähig werden.

Die Kläranlage kann wenigstens ein Nachklärbecken zwischen der aeroben Belebungseinrichtung und der Eindickungseinrichtung aufweisen, um die Klärung zu verbessern. Weiterhin kann das Nachklärbecken auch die Funktion eines Speichers übernehmen oder eine Pufferwirkung ausüben

Die aerobe Belebungseinrichtung ist vorzugsweise direkt mit dem Absetzbecken verbunden, was den Vorteil eines einheitlichen Leitungssystems und einer verminderten Geruchsbelästigung aufweist.

Die Fördervorrichtung bzw. Fördereinrichtung kann beispielsweise eine Rückschlammpumpe sein, welche vorzugsweise hinter dem Nachklärbecken angeordnet ist und mit einem Zulauf zu der aeroben Belebungseinrichtung verbunden ist. Damit kann die aerobe Aufbereitung des Abwassers weiter verbessert werden. Weiterhin kann die Fördereinrichtung beispielsweise auch eine Schaufelradeinrichtung sein. Auch die Fördereinrichtung für den eingedickten Schlamm zur anaeroben Behandlung kann mit einer Lysiereinrichtung ausgerüstet werden.

Vorzugsweise ist die Eindickungseinrichtung der Kläranlage eine Zentrifuge mit geeigneter Lysiereinrichtung. Dieses hat den Vorteil, daß neben der Lyse von Zellen eine wesentliche Entwässerung des Schlammes erzielt werden kann. Damit können ebenfalls die möglichen Transportkosten für den Schlamm erniedrigt werden. Ein weiterer Vorteil ist auch, daß der Wassergehalt des Schlammes eingestellt werden kann.

Weiterhin kann auch nach dem anaeroben Reaktor eine Eindickungs- und/oder Entwässerungseinrichtung, vorzugsweise eine Zentrifuge, angeordnet sein, um den Schlamm vor der Endlagerung und/oder Verbrennung möglichst stark zu trocknen. Auch hier bestehen die oben genannten Vorteile der verringerten Transportkosten sowie der Einstellbarkeit des Wassergehaltes.

In der Kläranlage wird wenigstens eine Vorrichtung zum Eindicken von Abwässerschlämmen mit Lysiereinrichtung verwendet, wie sie in den Ansprüchen 1 bis 42 beansprucht ist.

Die Lysiereinrichtung kann daher beispielsweise ein Friktionsmahlwerk, insbesondere Mahlscheiben- oder Mahlkonus-Mahlwerk, Profilreibe, Walzenquetsche, Passiertrommel, Schneidwerk oder ein Stiftmahlwerk umfassen.

Selbstverständlich sind auch Kombinationen dieser Lysiereinrichtungtypen in ein und derselben Zentrifuge und/oder Eindickvorrichtung denkbar.

Darüber hinaus können in der Kläranlage auch mehrere Zentrifugen und/oder Fördervorrichtungen mit jeweils unterschiedlichen Lysiereinrichtungen verwendet werden. Der Vorteil hiervon liegt darin begründet, daß die Abstimmung der Lysiereinrichtung auf die Schlammkonsistenz und -zusammensetzung durch solche Maßnahmen erfolgen kann.

Das aerob umgesetzte Abwasser kann in wenigstens einem Nachklärbecken nachgeklärt werden und das gereinigte Abwasser kann abgeleitet werden und wenigstens ein Teil des abgesetzten Klärschlammes kann in eine Eindickungsvorrichtung weitergeleitet werden. Dieses hat den Vorteil, daß weniger Volumen weiterverarbeitet werden muß, wodurch die Anlage ökonomischer wird.

Der anaerob umgesetzte Klärschlamm kann auch in einer weiteren Eindickungseinrichtung (Zentrifuge) eingedickt werden. Das gesamte Zentrat wird nach der Zentrifuge der aeroben Belebungseinrichtung zugeführt. Ein Teil des Zentrates von der Zentrifuge kann in den aeroben Reaktor zur Stimulierung geführt werden.

Vorzugsweise wird eine Menge von ca. 0,5 bis 50 % der Menge an ursprünglich anwesenden Organismenzellen lysiert. Damit kann die Stimulation und/oder die entstehende Schlammenge gesteuert werden.

Auch kann ein Teil des anaerob umgesetzten und/oder eingedickten Schlammes in den anaeroben Reaktor zurückgeführt werden, um ihn noch weiter abzubauen.

Zum Zerlegen (Destruktion) bzw. zur Lyse eines Teils der Zellen kann so beispielsweise die auf die Zellen beim Pumpen und/oder bei der Zentrifugation des verarbeiteten Materials (belebter oder anaerob stabilisierter Schlamm bzw. eine andere Biomasse) einwirkende Fliehkraft genutzt werden.

Ein weiterer Vorteil des mittels der Kläranlage durchgeführten erfindungsgemäßen Verfahrens ist, daß in dem anaeroben Reaktor ein brennbares Gas, insbesondere Methan, erzeugt wird. Das in dem Verfahren erzeugte, brennbare Gas kann zur Stromerzeugung verwendet werden, wobei vorzugsweise der erzeugte Strom direkt zum Betreiben der Kläranlage und/oder zur Einspeisung in das Stromnetz eingesetzt werden kann.

Weitere Vorteile und Merkmale der vorliegenden Erfindung ergeben sich aufgrund der Beschreibung von Ausführungsbeispielen sowie anhand der Zeichnung.

Es zeigt:
- Fig. 1: eine Gesamtansicht einer erfindungsgemäßen Vorrichtung im Längsschnitt;
- Fig. 2: eine,Schnittansicht eines Teils der erfindungsgemäßen Vorrichtung mit einer als Friktionsmahlwerk ausgebildeten Lysiereinrichtung gemäß einer ersten Ausführungsform;
- Fig. 3a: eine schematische Darstellung der Oberfläche einer Mahlscheibe, welche in der erfindungsgemäßen Vorrichtung gemäß einer ersten Ausführungsform eingesetzt wird;
- Fig. 3b: eine schematische Darstellung der Oberflächenvertiefungen gemäß Fig. 3a in einer anderen Ausgestaltung;
- Fig. 4: eine Schnittansicht eines Teils der erfindungsgemäßen Vorrichtung gemäß einer zweiten Ausführungsform;
- Fig. 5: eine Schnittansicht eines Teils der erfindungsgemäßen Vorrichtung gemäß einer dritten Ausführungsform;
- Fig. 6: eine Schnittansicht gemäß der Schnittlinie 6-6 in Fig. 5;
- Fig. 7: eine Schnittansicht eines Teils der erfindungsgemäßen Vorrichtung gemäß einer vierten Ausführungsform;
- Fig. 7a: eine Schnittansicht entlang der Linie 7-7 in Fig. 7;
- Fig. 8: eine Schnittansicht eines Teils der erfindungsgemäßen Vorrichtung gemäß einer fünften Ausführungsform;
- Fig. 9: eine Schnittansicht entlang der Linie 9-9 in Fig. 8;
- Fig. 10: eine Schnittansicht eines Teils der erfindungsgemäßen Vorrichtung gemäß einer sechsten Ausführungsform;
- Fig. 11: eine Schnittansicht entlang der Linie 11-11 in Fig. 10;
- Fig. 12: eine Schnittansicht entlang der Linie 12-12 in Fig. 10;
- Fig. 13: eine Schnittansicht eines Teils der erfindungsgemäßen Vorrichtung gemäß einer siebten Ausführungsform;
- Fig. 14: eine Schnittansicht entlang der Linie 14-14 in Fig. 13; und
- Fig. 15: eine schematische Darstellung einer Ausführungsform einen Kläranlage.

Im folgenden wird die erfindungsgemäße Vorrichtung beispielhaft anhand einer Zentrifuge mit einer Lysiereinrichtung beschrieben. Die Erfindung ist jedoch nicht hierauf beschränkt, sondern jede geeignete Fördereinrichtung für schlammhaltige Abwässer kann mit der beschriebenen Lysiereinrichtung kombiniert werden und an unterschiedlichen Positionen innerhalb einer Kläranlage im Sinne der vorliegenden Erfindung eingesetzt werden.

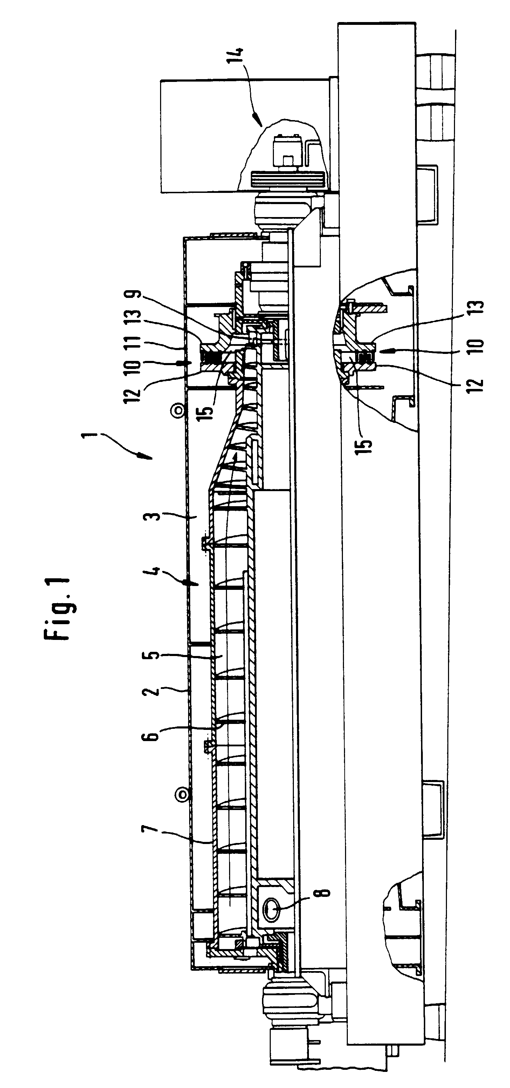

In Fig. 1 ist mit 1 eine Vorrichtung zum Eindicken von Abwasserschlämmen bezeichnet. Die Vorrichtung 1 weist ein Gehäuse 2 auf, in dessen Innerem 3 eine Zentrifuge 4 vorgesehen ist. Die Zentrifuge 4 weist in ihrem Inneren 5 einen Schneckenförderer 6 als rotierende Fördereinrichtung sowie einen rotierenden Mantel 7 auf.

Bei der Eindickung von Abwasserschlämmen wird Klärschlamm über eine nicht gezeigte Rohrleitung einem Zentrifugeneingang 8 der Zentrifuge 4 zugeführt. Der Abwasserschlamm wird dann durch den rotierenden Schneckenförderer 6 im Inneren 5 der Zentrifuge 4 in Richtung des in Fig. 1 gezeigten Pfeils zum hinteren Ende 9 und schließlich zum Zentrifugenausgang befördert. Im Beispielsfalle rotiert der Schneckenförderer 6 mit einer Umfangsgeschwindigkeit von ca. 3010 UPM und der Mantel 7 rotiert mit einer Umfangsgeschwindigkeit von etwa 3000 UPM, wobei die Rotationsrichtungen des Schneckenförderers 6 und des Mantels 7 identisch sind.

Am hinteren Ende 9 der Zentrifuge 4 ist im Beispielsfalle eine Lysiereinrichtung 10 innerhalb eines Gehäuses 11 angeordnet.

Die Lysiereinrichtung 10 umfaßt ein rotierendes Teil 12 sowie ein feststehendes Teil 13. Das rotierende Teil 12 der Lysiereinrichtung 10 sowie der Mantel 7 der Zentrifuge 4 werden über einen Antrieb 14 angetrieben.

Der Schneckenförderer 6 der Zentrifuge 4 wird über einen zweiten, in Fig. 1 nicht näher dargestellten, Antrieb, z.B. hydraulisch oder über ein Getriebe, angetrieben.

Zur Gewinnung von Lysatschlamm wird der bei Kläranlagen üblicherweise anfallende Klärschlamm über den Schneckenförderer 6 der Zentrifuge 4 zum Zentrifugenausgang 9 gefördert, um in das Innere 15 der Lysiereinrichtung 10 zu gelangen. Zellen von Organismen, insbesondere Mikroorganismen, wie Protozoen und Bakterien, sowie Algen und Nematoden, jedoch auch Bestandteile höherer Pflanzen, werden in der Lysiereinrichtung 10 derart zerstört bzw. lysiert, daß deren Membranen und/oder Zellwände zerreißen und sich der Zellinhalt in die Umgebung ergießt.

Da naturgemäß nicht sämtliche in dem Schlamm vorhandenen Zellen lysiert werden, dient der Zellinhalt der lysierten Zellen anderen Organismen bei der klärtechnischen Weiterverarbeitung des Schlammes als Nährmedium, wodurch zum einen die Biogasproduktion, insbesondere Methanproduktion im Faulturm, in erheblichem Maße zunimmt, und zum anderen die Gesamtschlammasse in dramatischem Maße abnimmt.

Im folgenden werden nun Ausführungsformen der erfindungsgemäßen Vorrichtung mit unterschiedlichen Lysiereinrichtungen 10 beschrieben.

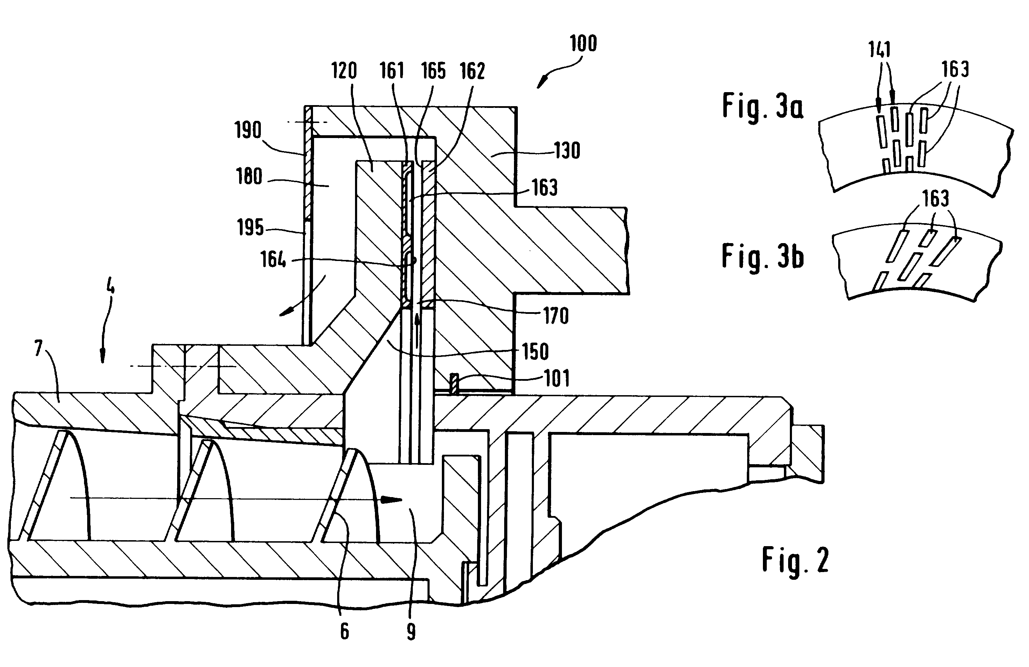

Fig. 2 zeigt eine Lysiereinrichtung 100, welche am Zentrifugenausgang 9 der Zentrifuge 4 einer Vorrichtung 1 zum Eindicken von Abwasserschlämmen angeordnet ist.

Das rotierende Teil 120 der Lysiereinrichtung 100 gemäß Fig. 2 ist mit dem Mantel 7 der Zentrifuge 4 verbunden, so daß es mit dem Mantel 7 rotiert. Das feststehende Teil 130 der Lysiereinrichtung 100 ist als stehende Tasse ausgebildet. In der Ausführungsform gemäß Fig. 2 weist die Lysiereinrichtung 100 die Form eines Friktionsmahlwerkes auf einem Teil der Oberfläche des rotierenden Teiles 120 eine Mahlscheibe 161 auf. Das feststehende Teil 130 der Lysiereinrichtung 100 weist ebenfalls eine Mahl scheibe 162 auf.

Im Beispielsfalle weist die Mahlscheibe 161 Vertiefungen 163 auf, welche vorzugsweise gemäß Fig. 3a in radialer Richtung ausgerichtet sind. Aufgrund der Rotation des Schneckenförderers 6 und des Mantels 7 der Zentrifuge 4 wird Abwasserschlamm in Pfeilrichtung zum Zentrifugenausgang 9 gefördert und mit einer Geschwindigkeit von ca. 50 m/s in das Innere 150 der Lysiereinrichtung 100 transportiert. Der Schlamm muß nun durch den Spalt 170 hindurchtreten, wo er zwischen den Mahlflächen 164 und 165 vermahlen wird. Dabei sind die Vertiefungen 163, welche vorzugsweise in der rotierenden Mahlscheibe 161 angebracht sind, von besonderem Vorteil, da sie die Mahl- und Scherkräfte der Lysiereinrichtung 100 verstärken und somit zu einer erhöhten Zellyse führen.

Nachdem der Schlamm im Spalt 170 vermahlen wurde, sammelt er sich aufgrund der Zentrifugalkraft am in Fig. 2 oberen Ende 180 der Lysiereinrichtung 100 an. Die Schlammhöhe und damit die gesamte Verweildauer des Schlammes in der Lysiereinrichtung 100 ist zum einen über die Breite des Spaltes 170, welche im Beispielsfalle ca. 2 mm beträgt, sowie durch die Höhe eines Wehrringes 190 bestimmt.

Nach dem Passieren der Lysiereinrichtung 100 tritt der Schlamm in eingedickter Form am Ausgang 195 der Lysiereinrichtung 100 aus.

Gemäß Fig. 3a und 3b sind die Vertiefungen 163 auf der Oberfläche der rotierenden Mahl fläche 161 vorzugsweise als Nuten in Radialrichtung ausgebildet, können jedoch gemäß Fig. 3b auch einen Anstellwinkel gegenüber der Radialrichtung aufweisen.

Im Beispielsfalle ist es besonders bevorzugt, daß die Vertiefungen 163 auf Lücke angeordnet sind. Dies bedeutet, daß nebeneinanderliegende Nutenreihen 141 so angeordnet sind, daß Bereiche, an denen eine Nutenreihe 141 unterbrochen ist, die benachbarten Nutenreihen 141 keine Unterbrechung aufweisen.

Der Vorteil liegt einerseits in einer erhöhten pumpenden Wirkung und einer Ausbildung von größeren Druckgradienten innerhalb des Spaltes 170, so daß sich insgesamt eine deutlich erhöhte lysierende Wirkung der Lysiereinrichtung 100 ergibt.

Allerdings kann die Lysiereinrichtung gemäß Fig. 2 auch mehrere Lysiereinrichtungen 100 umfassen, so daß eine mehrstufige Lysiereinrichtung zur Verfügung steht, wodurch sich die lysierende Wirkung noch deutlich erhöht.

Selbstverständlich ist die Zahl der Lysiereinrichtungsstufen durch den erforderlichen Energieaufwand und das Verhältnis zur Biogasgewinnung, also mit anderen Worten, das Energieaufwand/Nutzen-Verhältnis, limitiert.

Darüber hinaus kann zum Zurückhalten von größeren Fremdkörpern, welche gegebenenfalls die Lysiereinrichtung 100 beschädigen könnten, eine Siebstufe vor Eintritt des Schlammes in den Spalt 170 oder auch vor dem Zentrifugeneingang 8 angeordnet werden.

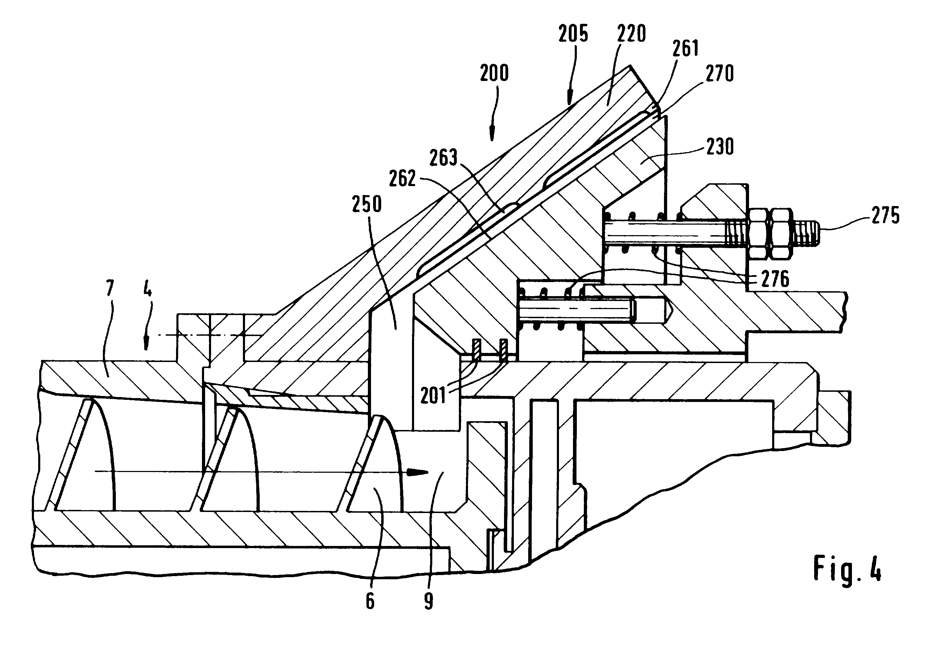

In Fig. 4 ist mit 200 die Lysiereinrichtung einer erfindungsgemäßen Vorrichtung 1 in einer zweiten Ausführungsform dargestellt. Die Lysiereinrichtung 200 ist in diesem Fall als Friktionsmahlwerk, und zwar als Mahlkonus 205 ausgebildet. Der rotierende Außenkonus 220 des Mahlkonus 200 ist mit dem Mantel 7 der Zentrifuge 4 verbunden. Die Mahlfläche 261 des Außenkonus 220 weist Vertiefungen 263 auf. Im Beispielsfalle bevorzugt sind solche Vertiefungen 263, welche in Richtung der Mantellinien des Mahlkonus 205 ausgerichtet sind. Grundsätzlich ist es jedoch auch möglich, die Vertiefungen 263 in einem Anstellwinkel zu den Mantellinien des Mahlkonus 205 anzuordnen. Im Beispielsfalle sind die als Nuten ausgebildeten Vertiefungen 263 auf der Mahlfläche 261 des Außenkonus 220 so angeordnet, daß nebeneinanderliegende Nutenreihen auf Lücke angeordnet sind, so daß Bereiche, an denen eine Nutenreihe unterbrochen ist, die benachbarten Nutenreihen keine Unterbrechung aufweisen.

Hierdurch wird ebenfalls eine bessere Lysierwirkung erreicht.

Dem rotierenden Außenkonus 220 gegenüber liegt ein feststehender Innenkonus 230, welcher eine Mahlfläche 262 aufweist. Zwischen Innenkonus 230 und Außenkonus 220 der Lysiereinrichtung 200 liegt ein Spalt 270, dessen Breite über eine Stelleinrichtung 275 verändert werden kann.

Zur Verbesserung der Pumpwirkung und der besseren Verteilung des Schlammes wird im Beispielsfalle die Tiefe der Vertiefungen 263 so gewählt, daß sie annähernd mit der Breite der Vertiefungen 263 übereinstimmt. Besonders vorteilhaft an einem Mahlkonus 205 als einer Lysiereinrichtung 200 einer Vorrichtung 1 zum Eindicken von Abwasserschlämmen liegt darin begründet, daß eine derartige Lysiereinrichtung 200 in Form des Mahlkonus 205 Fremdkörpern, welche in dem Schlamm in das Innere 250 des Mahlkonus 205 eintreten, nicht zum Zerstören des Mahlkonus 205 führen, sondern aufgrund einer gefederten Lagerung 276 aufgenommen werden können, so daß eine Abtrennung von Fremdkörpern mittels eines Siebes nicht unbedingt erforderlich ist.

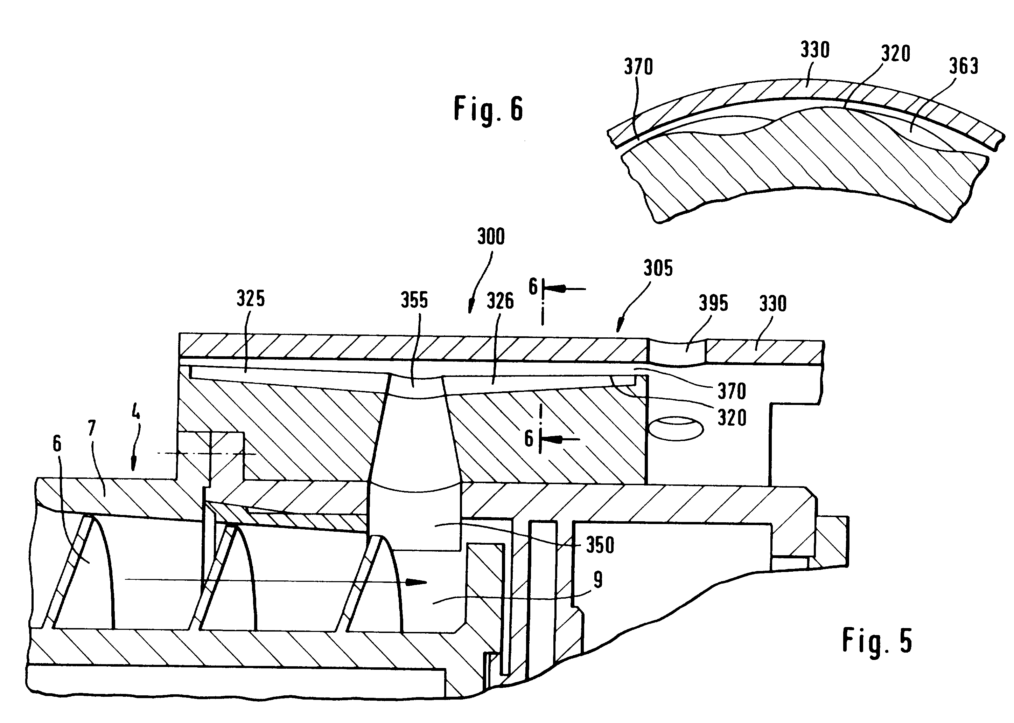

Fig. 5 zeigt als Lysiereinrichtung 300 eine Profilreibe 305 als dritte Ausführungsform der vorliegenden erfindungsgemäßen Vorrichtung 1. Die Profilreibe 305 weist eine rotierende Reibfläche 320 und eine feststehende Außenhülle 330 auf. Die rotierende Reibfläche 320 ist mit dem Mantel 7 der Zentrifuge 4 verbunden. Gemäß Fig. 5 wird Schlamm in Pfeilrichtung durch den Schneckenförderer 6 sowie den rotierenden Mantel 7 in das Innere 350 der Profilreibe 305 befördert und tritt aus einer Austrittsöffnung 355 in den zwischen der rotierenden Reibfläche 320 und der feststehenden Reibfläche 330 gebildeten Spalt 370 ein und wird dort gequetscht und zerrieben, so daß in dem Schlamm enthaltene Zellen aufgrund der hohen Scherkräfte und des hohen Druckes innerhalb des Spaltes 370 lysiert werden, wodurch sich ihr Zellinhalt in das umgebende Medium ergießt.

Zur Erhöhung des Druckes innerhalb des Spaltes 370 ist im Beispielsfalle der vom Ausgang 395 abgewandte Teil 325 der rotierenden Reibfläche 320 stärker zu der Außenhülle 330 geneigt und der der Ausgangsöffnung 395 zugewandte Teil 326 der rotierenden Reibfläche 320 weniger stark zu der feststehenden Reibfläche 330 geneigt, so daß sich in diesem Bereich ein breiterer Spalt 370 ergibt als auf der anderen Seite der Austrittsöffnung 355. Dieses nach außen ansteigende Niveau erzeugt eine beachtliche Druckerhöhung, welche wiederum die Lysierwirkung der Lysiereinrichtung 300 verstärkt.

Bei dieser Ausführungsform ist es vorteilhaft, daß praktisch keine Dichtigkeitsprobleme auftreten, die Ausführungsform der Lysiereinrichtung einfach zu bauen ist, keine Metallreibung auftritt und das größere Teilchen seitlich ausweichen können, so daß in der Regel keine zusätzlichen Siebmaßnahmen erforderlich sind.

Fig. 6 zeigt einen Schnitt entlang der Linie 6-6 in Fig. 5. Gemäß dieser Ausführungsform der Lysiereinrichtung 300 weist die Profilreibe 305 auf ihrer rotierende Reibfläche 320 wellenförmige Vertiefungen 363 auf, welche bevorzugt einen Anstellwinkel zur Drehrichtung aufweisen. Im Beispielsfalle beträgt die Breite des Spaltes 370 an seiner engsten Stelle ca. 2 mm und an seiner breitesten Stelle ca. 10 mm.

Bei dieser Ausführungsform wird der Schlamm dazu gezwungen, durch den engen Abstand zwischen der feststehenden Reibfläche 330 und der rotierenden Reibfläche 320, welche wellenförmige Vertiefungen 320 aufweist, zu fließen. Hierdurch entsteht ein hoher Druck im Spalt 370 und läßt so hohe Scherkräfte entstehen, daß Membranen und Zellwände der Mikroorganismen, insbesondere Bakterien, zerreißen und sich ihr Zytoplasma in das umgebende Medium entleert.

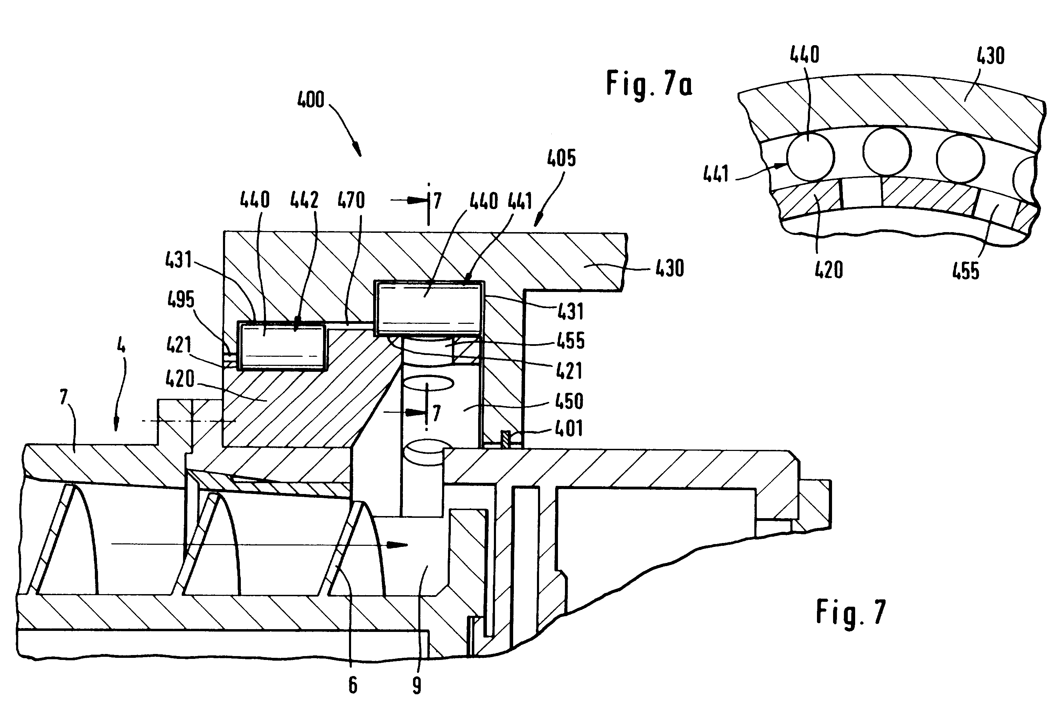

Fig. 7 zeigt als Lysiereinrichtung 400 eine Walzenquetsche 405 als vierte Ausführungsform der vorliegenden Erfindung.

Das rotierende Teil 420 der Walzenquetsche 405 ist wiederum mit dem Mantel 7 der Zentrifuge 4 verbunden, so daß es mit diesem rotiert. Die Lysiereinrichtung 400 wird nach außen über ein feststehendes Teil 430 abgeschlossen, weist jedoch zwischen rotierendem Teil 420 und zwischen feststehenden Teil 430 eine Auslaßöffnung 495 auf.

Sowohl das rotierende Teil 420 als auch das feststehende Teil 430 weisen Ausnehmungen 421 und 431 auf. In den Ausnehmungen 421 und 431 sind Rollkörper, im Beispielsfalle Walzen 440 vorgesehen. Im Beispielsfalle weist die Lysiereinrichtung 400 zwei Walzensätze 441 und 442 auf.

Bei Rotation des rotierenden Teiles 420 der Walzenquetsche 405 bewegen sich die Walzen 440 nach Art eines Wälzlagers zwischen dem rotierenden Teil 420 und dem feststehenden Teil 430.

Aufgrund der Bewegung des Mantels 7 und des Schneckenförderers 6 in Pfeilrichtung gemäß Fig. 7, tritt Schlamm am Zentrifugenausgang 9 der Zentrifuge 4 in das Innere 450 der Walzenquetsche 405 als Lysiereinrichtung 400 ein. Der organismenhaltige Klärschlamm tritt dann durch die Austrittsöffnung 455 zwischen zwei Walzen 440, so daß die WaLzen 440 im Spiel zwischeneinander Schlamm erfassen, überrollen und dabei derart quetschen, daß die darin vorhandenen Zellen lysiert werden.

Der derart zerquetschte und lysierte Schlamm wird dann über den Spalt 470 weiter zum nächsten Walzensatz 442 transportiert, wo er erneut durch die umlaufenden Walzen 440 überrollt und gequetscht wird, so daß noch mehr Organismenzellen zerstört werden, so daß sich deren Zellinhalt in das Umgebungsmedium ergießt.

Der mit der Walzenquetsche 405 behandelte Schlamm verläßt schließlich die Lysiereinrichtung 400 an ihrer Auslaßöffnung 495, wo der Schlamm dann, je nach Bedarf, weiterverarbeitet wird.

Besondere Vorteile der vorliegenden beispielhaften vierten Ausführungsform einer Vorrichtung 1 zum Eindicken von Abwasserschlämmen gemäß Fig. 7 liegen darin begründet, daß unterschiedliche Spaltbreiten 470 eingestellt werden können und unterschiedliche Walzengrößen benutzt werden können, je nachdem, welche lysierende Wirkung man erzielen möchte, so daß durch Variation der Parameter Breite des Spaltes 470, Größe der Walzen 440 und Anzahl der Walzensätze 441, 442 eine Feineinstellung des Lysierungsgrades erreicht werden kann.

Selbstverständlich ist es auch möglich, in Fig. 7 nicht gezeigte Vertiefungen in die Oberflächen der Walzen 440 einzubringen, welche sowohl radial, als auch in Richtung der Mantellinien der Walzenzylinder wie auch mit einem Anstellwinkel zur Drehrichtung vorgesehen sein können.

In Fig. 7a ist ein Schnittbild entlang der Linie 7-7 in Fig. 7 durch einen ersten Walzensatz 441 und die Austrittsöffnung 455 gezeigt.

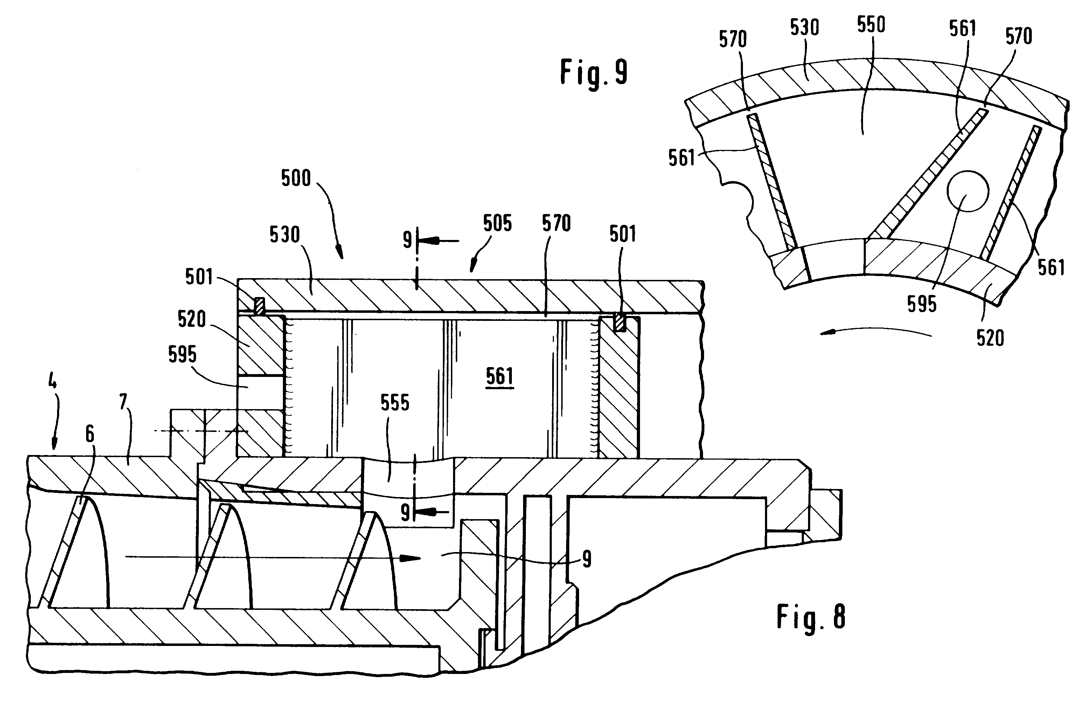

In Fig. 8 ist als Lysiereinrichtung 500 eine Passiertrommel 505 gemäß einer fünften Ausführungsform der vorliegenden Erfindung gezeigt.

Die Passiertrommel 505 weist ein rotierendes Teil 520 und ein feststehendes Teil 530 auf, welche mittels Dichtungen 501 gegeneinander abgedichtet sind.

Im Inneren 550 der Lysiereinrichtung 500 sind Passierelemente 561, im Beispielsfalle in unterschiedlichen Winkeln zueinander angeordnet.

Wie in den anderen Ausführungsformen ist das rotierende Teil 520 mit dem Mantel 7 der Zentrifuge 4 verbunden.

Aufgrund der Bewegung des Schneckenförderers 6 sowie des Mantels 7, wird Schlamm in Pfeilrichtung zum Zentrifugenausgang 9 befördert und tritt durch die Austrittsöffnung 555 in das Innere 550 der Passiertrommel 505. Schlamm und darin enthaltene Mikroorganismen, wie beispielsweise Bakterien, werden durch den engen Spalt 570 zwischen feststehendem Teil 530 und den Passierelementen 561 gequetscht, wodurch hohe Scherkräfte entstehen, so daß Bakterien und andere Mikroorganismen durch diese Scherbeanspruchung zerrissen werden und hierdurch lysiert werden, wodurch sie ihren Zellinhalt in das umgebende Medium ergießen.

Der in der Lysiereinrichtung 500 behandelte Schlamm tritt an der Auslaßöffnung 595 aus und kann in geeigneter Form weiter verarbeitet werden.

Fig. 9 zeigt einen Schnitt durch die Passiertrommel 505 gemäß der Linie 9-9 in Fig. 8.

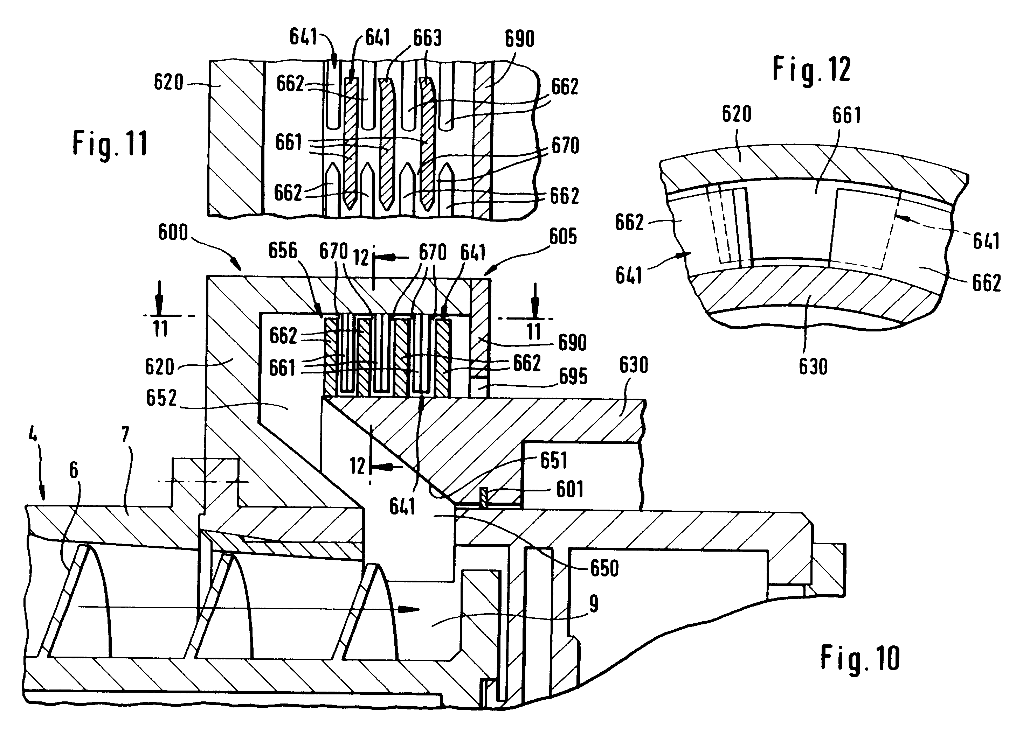

Fig. 10 zeigt als Lysiereinrichtung 600 ein Schneidwerk 605 als sechste Ausführungsform der vorliegenden Vorrichtung 1 zum Eindicken von Abwasserschlämmen. Das Schneidwerk 605 weist ein rotierendes Teil 620 auf, welches Schneidelemente 661 trägt.

Das rotierende Teil 620 ist mit dem Mantel 7 der Zentrifuge 4 verbunden. Das feststehende Teil 630 der Lysiereinrichtung 600 weist Schneidelemente 662 auf, welche in die Schneidelemente 661 eingreifen. Im Beispielsfalle sind die Schneidelemente als Messer ausgebildet, wobei die Messer sowohl hintereinander als auch nebeneinander angeordnet sind, wobei vorzugsweise eine Anordnung gemäß Fig. 12, die einen Schnitt entlang der Linie 12-12 in Fig. 10 darstellt, auf Lücke angeordnet sind.

Die Rotation des rotierenden Teiles 620 des Schneidwerks 605 erfolgt derart, daß sich die einzelnen Schneidelemente 661 und 662 nicht berühren und zwischen ihnen Spalte 670 ausgebildet sind, welche bei Bedarf einstellbar sind.

Wie in den anderen Ausführungsformen wird Schlamm aus der Zentrifuge 4 zum Zentrifugenausgang 9 befördert, welcher mit einer Geschwindigkeit von ca. 50 m/s in das Innere 650 der Lysiereinrichtung 600 befördert wird.

Der Schlamm aus der Zentrifuge 4 trifft dann zunächst auf die schräge Oberfläche 651 des feststehenden Teils 630 auf. Aufgrund dieses Aufschlagens des Klärschlammes platzten bereits hier eine Vielzahl der vorhandenen Zellen von Mikroorganismen, wie Bakterien und Protozoen.

Um die lysierende Aufprallwirkung auf die schräge Oberfläche 651 noch zu verstärken, können hierauf noch Unebenheiten, etwa in Form von Messern, Stiften oder dergleichen angebracht sein.

Der Schlamm wird dann weiter über den Kanal 652 zu der eigentlichen Schneideeinrichtung 656 geführt. Die als Messer ausgebildeten Schneidelemente 661 rotieren im Beispielsfalle mit einer Umfangsgeschwindigkeit von ca. 80 m/s. Der Schlamm wird dann durch die Spalte 670 zwischen den Messern 661 und 662 hindurchgeführt und muß im vorliegenden Beispielsfall vier Reihen von Schneidelementen 662 passieren, um schlußendlich zum Schlammauslaß 695 zu gelangen.

Aufgrund der Rotationsgeschwindigkeit sowie der Ausgestaltung der Schneideeinrichtung 656 und der Schneidelemente 661 und 662 treten enorme Scherkräfte an den im Schlamm enthaltenen Mikroorganismen auf, so daß der größte Teil der darin enthaltenen Mikroorganismenzellen zerrissen wird, wodurch sich eine extreme lysierende Wirkung des Schneidwerks 605 ergibt, wodurch der größte Anteil der im Schlamm enthaltenen Zellen lysiert wird und sich deren Cytoplasma in das umgebende Medium ergießt, und für überlebende Zellen ein ausgezeichnetes Nährmedium bildet, was dann zu erhöhter Biogasproduktion und verminderter Schlammenge führt.

Fig. 11 zeigt einen Schnitt entlang den Linien 11-11 in 5 Fig. 10, aus welchem erkennbar ist, daß in der Lysiereinrichtung 600 auch Schneidelemente 661 oder 662 verwendet werden können, deren Ende, im Beispielsfalle ein Messerende 663, so ausgestellt ist, daß ein Teil des Schlammes der vorherigen Messerreihe wieder zugeführt wird, wodurch sich 10 eine gewisse Pumpwirkung in Richtung auf die Zentrifuge ergibt und wodurch die Scherkräfte und damit die lysierende Wirkung noch weiter gesteigert werden kann.

Am Auslaß 695 befindet sich ein Wehr 690, mit dessen Höhe 15 die Verweilzeit innerhalb der Schneideinrichtung 656 und damit der Lysierungsgrad einstellbar ist.

Fig. 12 zeigt einen Schnitt entlang der Linie 12-12 in Fig. 10, aus dem ersichtlich ist, daß die Schneidelemente 661 20 und 662, welche in Schneidelemente- oder Messerreihen 641 angeordnet sind, auf Lücke zueinander stehen.

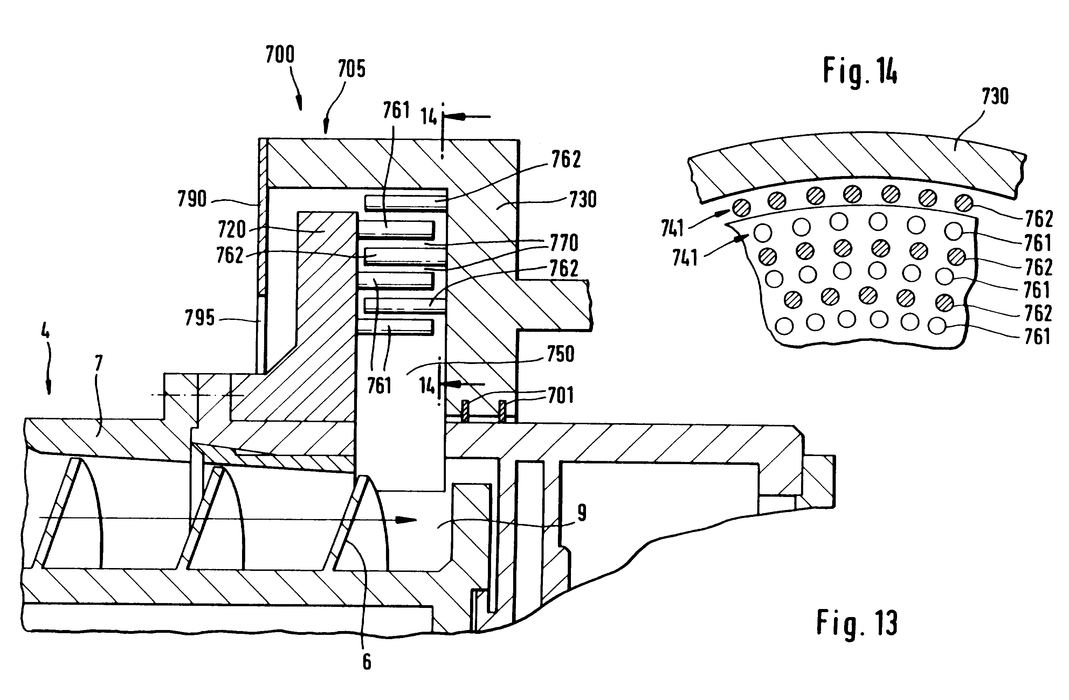

Fig. 13 zeigt eine Schnittansicht eines Stiftmahlwerkes 705 einer Lysiereinrichtung 700 einer erfindungsgemäßen Vorrichtung 1 in einer siebten Ausführungsform.

Das Stiftmahlwerk ist wie die anderen Ausführungsformen als integraler Bestandteil der Zentrifuge 4 am Zentrifugenausgang 9 angeordnet. Die Lysiereinrichtung 700 und insbesondere das Stiftmahlwerk 705, weist ein rotierendes Teil 720 und ein feststehendes Teil 730 auf. Das feststehende Teil 730 ist nach außen über Dichtungen 701 abgedichtet.

Das rotierende Teil 720 ist mit dem Mantel 7 der Zentrifuge 4 verbunden. Das rotierende Teil 720 des Stiftmahlwerkes 705 trägt mehrere Reihen von Stiften 761, welche in die Lücken, die von Stiften 762 gebildet werden, und die an dem feststehenden Teil 730 vorgesehen sind, eingreifen.

Im Beispielsfalle weist das rotierende Teil 720 und das feststehende Teil 730 jeweils drei Stiftreihen 741 auf.

Das Stiftmahlwerk 705 ist nach außen über ein Wehr 790 abgeschlossen und weist einen Schlammauslaß 795 auf.

Wie in den anderen Ausführungsformen wird Schlamm in Pfeilrichtung durch die Zentrifuge 4 zum Zentrifugenausgang 9 befördert und tritt dann in das Innere 750 der Lysiereinrichtung ein. Der Schlamm wird dann in die Zwischenräume 770 zwischen den einzelnen Stiften 761 und 762 gezwungen und wird durch Rotation der Stifte 761 großen Scherkräften ausgesetzt. Ähnlich wie beim Schneidwerk 605 der Lysiereinrichtung 600 treten in dem Stiftmahlwerk 705 zwischen den einzelnen Stiften 761 und 762 bzw. zwischen den einzelnen Stiftreihen 741 große Scherkräfte auf, welche in der Lage sind, Zellen von im Schlamm enthaltenen Mikroorganismen zu zerstören, so daß sich deren Inhalt in das umgebende Medium ergießt.

Der mit der Lysiereinrichtung 700 behandelte Schlamm tritt dann am Schlammauslaß 195 aus und kann dann wie gewünscht weiterverarbeitet werden.

Die Verweilzeit des Schlammes und damit der Lysiergrad kann durch die Höhe des Wehrs 790 eingestellt werden.

In einer bevorzugten Ausführungsform des vorliegenden Stiftmahlwerks 705 ist der Abstand zwischen zwei Stiftreihen 741 einstellbar, wodurch sich noch größere Scherkraftwirkungen ergeben und damit höhere Lysiergrade erzielt werden.

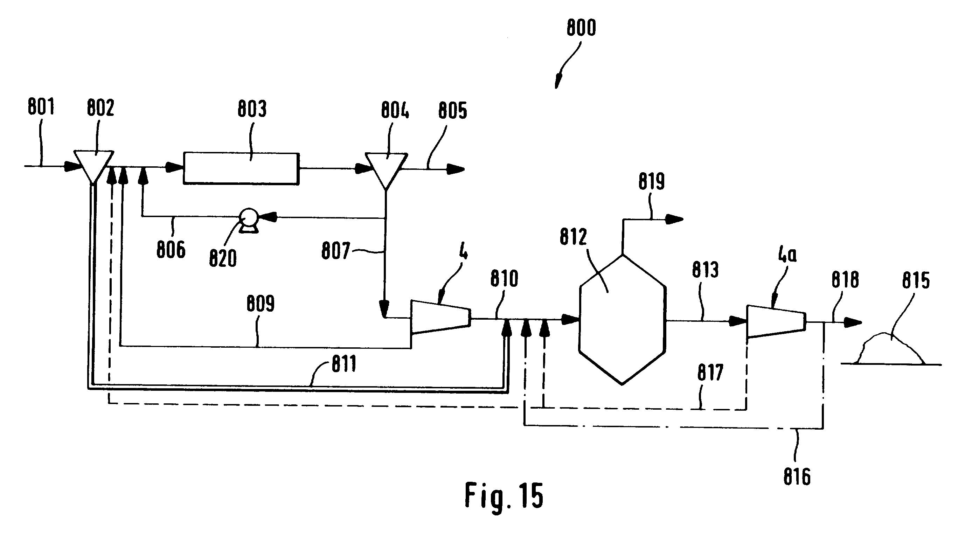

Fig. 14 zeigt einen Schnitt entlang der Linie 14-14 in Fig. 13.

Besondere Vorteile der Lysiereinrichtung 700 in Form eines Stiftmahlwerkes 705 liegen darin begründet, daß die Verweilzeit einstellbar ist, die Effezienz beispielsweise durch die Zahl der Stifte 761 und 762 leicht variierbar ist und daß die einzelnen Stifte 761, 762 im Bedarfsfalle leicht austauschbar sind.

Ein weiterer Vorteil liegt in dem relativ kleinen Bauraum und in relativ großen Toleranzen für die einzelnen Stifte. Bei Bedarf ist es sinnvoll, am Zentrifugenausgang 9 oder im Zulauf der Zentrifuge 4 ein in Figur 13 nicht gezeigtes Sieb anzuordnen, um größere Fremdkörper, welche eventuell die Stifte 761 und 762 beschädigen könnten, zurückzuhalten.

Neben den hohen Scherkräften, welche zwischen den Stiftreihen 741 auftreten, wird die lysierende Wirkung durch Aufprallen und Zerschlagen der Zellen ermöglicht.

Die in Fig. 15 gezeigte Kläranlage zur Anwendung eines Abwasserklärverfahrens umfaßt ein primäres Absetzbecken 802 mit einem Zufluß 801 von rohem Abwasser. Ein Primärschlamm 811 wird in einen anaeroben Reaktor 812 geleitet. Das Abwasser aus dem primären Absetzbecken 802 wird in eine aerobe biologische Belebungseinrichtung 803 geführt, die Mischung aus der Belebungseinrichtung 803 wird in ein Nachklärbecken 804 geleitet, wo es zur Trennung des gereinigten Abwassers 805 kommt. Ein Teil des abgesetzten belebten Schlammes 806 wird mit einer Rückschlammpumpe 820 als Fördereinrichtung in die Belebungseinrichtung 803 zurückgepumpt. Der überschüssige belebte Schlamm 807 wird in eine Eindickungszentrifuge 4 mit Lysiereinrichtung 1O geführt, wo es zur Schlammeindickung sowie zur Zerlegung von Zellen eines Teils der Mikroorganismen kommt. Ein Zentrat 809 gelangt in die Belebungseinrichtung 803 zurück. Der eingedickte Schlamm wird in den anaeroben Reaktor 812 geführt. Eine Reaktionsmischung 813 vom anaeroben Reaktor 812 wird in eine Eindickungs- oder Entwässerungszentrifuge 4a geleitet, wo es zur Entwässerung des stabilisierten Schlammes und zur Zerlegung von Zellen eines Teils der Mikroorganismen kommt. Ein Zentrat 817 gelangt in die Belebungseinrichtung 803 zurück und/oder ein Teil davon wird dem anaeroben Reaktor 812 zugeleitet. Der entwässerte anaerob stabilisierte Schlamm 815 gelangt über einen Auslaß 818 auf eine Deponie und/oder ein Teil 816 davon wird in den anaeroben Reaktor 812 zurückgeführt.

Der Teil dieser Anlage, wo es zur teilweisen Zerstörung der Zellen der Mikroorganismen kommt, setzt sich aus der Eindickungszentrifuge 4 und der Entwässerungszentrifuge 4a und/oder der Rückschlammpumpe 820 und der o.a. Schlammpumpe zusammen. Im Beispielsfalle ist als Lysiereinrichtung ein Friktionsmahlwerk 100 mit einer rotierenden Mahlscheibe 161 und einer feststehenden Mahlscheibe 162 bei Zentrifuge 4 vorgesehen, während am Ausgang 9 der Zentrifuge 4a eine Lysiereinrichtung 10 in Form eines Messerschneidwerks 605 vorgesehen ist.

Zur Aufbereitung des Stimulierungsreagens ist es auch möglich, einerseits belebten Schlamm und andererseits anaerob stabilisierten Schlamm und zwar entweder direkt aus dem gegebenen Reaktor oder aus einem anderen, gut arbeitenden Reaktor, zu nutzen. Im erstgenannten Fall wird der überschüssige belebte Schlamm 807 in die Eindickungszentrifuge 4 geführt, wo es außer der Schlammeindickung zur Zerlegung/Lyseeines Teils der Biomassenzellen in der Lysiereinrichtung kommt, wobei das Zentrat 809 in die Belebungseinrichtung 803 zurückkehrt und der eingedickte Teil 810 in den anaeroben Reaktor 812 zur Schlammstabilisierung geführt wird. Im anderen Fall wird der anaerob stabilisierte Schlamm 813 in die Entwässerungszentrifuge 4a geführt, wo es außer der Schlammentwässerung zum Abbau eines Teils der Biomassenzellen kommt, wobei ein Teil von 5 bis 30 % des Zentrates 817 und/oder ein Teil des zu 5 bis 30 % entwässerten Schlammes 816 in den anaeroben Reaktor 812 zur Schlammstabilisierung zurückgeführt wird.

Das mit der erfindungsgemäßen Kläranlage durchgeführte Verfahren ist somit ökonomisch deutlich vorteilhafter aus der Sicht einer Nutzung in industriellem Maßstab im Vergleich zu dem Stand der Technik der separaten Lysatzubereitung. Auf diese Weise ist es möglich, gleichzeitig die aeroben und anaeroben biologischen Klärprozesse zu stimulieren, hierdurch die zu entsorgende Schlammenge zu verringern und die Biogasproduktion zu erhöhen.

Eine Anwendung der erfindungsgemäßen Vorrichtung bzw. Kläranlage wirkt sich bei ihrer Realisierung allgemein durch eine Verbesserung einer Reihe von technischen Parametern aus. Bei der Verarbeitung gelöster Verunreinigungen (anaerobe Abwasserreinigung), sowie der anaeroben Schlammstabilisierung kommt es zu folgenden Ergebnissen: Die Leistung der anaeroben Reaktoren wird erhöht, der Abbau organischer Stoffe wird beschleunigt, der Abbau organischer Stoffe im Verlauf des Stabilisierungsprozesses wird ebenfalls beschleunigt (bei Schlamm die Vertiefung des anaeroben Abbaus, beim Abwasser die Möglichkeit, Problemstoffe, wie z.B. verschiedene Xenobiotika oder Giftstoffe abzubauen), die Biogasproduktion wird erhöht, die Produktion von stabilisierendem Schlamm wird vermindert, die Entwässerungsfähigkeit des anaerob stabilisierten Schlammes wird verbessert und die Energiebilanz des Prozesses im Vergleich zur herkömmlichen Gestaltung wird ebenfalls verbessert.

### Beispiel 1

Die Funktion sowie die technische Nutzung laut Erfindung ist der Fig. 15 zu entnehmen. Als Hauptanlage zur Zubereitung des Stimulierungsreagens, d.h. zum Abbau oder der Lyse eines Teils der Biomassenzellen des überschüssigen belebten Schlammes wurde eine Eindickungszentrifuge 4 verwendet. Zur Ermittlung der Menge des freigewordenen Zellysats wurde die Konzentration der gelösten organischen Stoffe, ausgedrückt als BSB₅ im Eingangsstrom 807 und im Ausgangsstrom 810 aus der Eindickungszentrifuge 4 mit folgendem Ergebnis festgelegt:

| | | |
|---|---|---|
| Eingangsstrom (807) | BSB_{5(gelöst)} | 140 mg/l |
| Konzentrat (Strom 810) | BSB_{5(gelöst)} | 630 mg/l |

Mit Eingangsstrom 807 und Konzentrat 810 wurden die Tests der methanogenen Aktivität durchgeführt. Die Konzentrationen der suspendierten Stoffe wurden bei beiden Stromarten so gestaltet, daß sie gleich waren. Getestet wurden unter gleichen Bedingungen und mit derselben Inokulum-Konzentration. Als Inokulum wurde anaerob stabilisierter Schlamm aus einem anaeroben Reaktor eingesetzt. Ermittelt wurde die Biogasproduktion getrennt für jeden Strom und für die Mischung beider Ströme mit gleicher Menge sämtlicher Stoffe. Die Biogasproduktion aus derselben Menge sämtlicher Stoffe war beim Konzentrat 810 um ca. 10,1 % höher als beim Eingangsstrom 807. Das Produktionsergebnis bei der Mischung ergab eine Erhöhung um ca. 13,3 % und ca. 31,2 % gegenüber dem theoretischen Wert in Abhängigkeit von der Belastung des anaeroben Inokulums (0,54 und 0,27 g CSB/g organischer Anteil in einem Gramm der nicht aufgelösten Stoffe (Glühverlust)). Der theoretische Wert stellt die Summe des Produktionsgaswertes derselben Menge für jeden Strom einzeln dar.

### Beispiel 2

### Überprüfung des Stimulierungseinflusses des Zellysates auf den anaeroben Abbau mit einfachen Substraten.

Es wurden methanogene Aktivitätstests anaerober Kofermentation mit Ameisensäure, Essigsäure, Propionsäure und Glukose vorgenommen. In sämtlichen Fällen wurde die gleiche Inokulum-Menge eingesetzt, eine Versuchsserie wurde unter Zugabe von eingedicktem überschüssigem belebtem Schlammkonzentrat (Strom 810) durchgeführt und die andere unter Zugabe der gleichen Menge eintretenden überschüssigen belebten Schlammes vor der Zentrifugierung (Strom 807). Die Kofermentation der komplexen Materialien mit einfachen Substraten hat manchmal eine Erhöhung der Abbaufähigkeit einiger Komponenten der komplexen Substrate zur Folge. Die Kofermentation der gleichen Menge der untersuchten Schlämme mit Glukose wies einen Anstieg der Abbau fähigkeit beim eintretenden Schlamm um ca. 41,8 % und beim Konzentrat um ca. 51,3 % auf (Unterschied in der Auswirkung ca. 11,3 %). Die Kofermentation mit Ameisensäure war nur bei ca. 13,5 %-Konzentrat positiv (Unterschied in der Auswirkung ca. 33 %).

## Patentansprüche

1. Eindickzentrifuge zum Eindicken von Überschußschlamm (807),
**dadurch gekennzeichnet, daß**
an der Eindickzentrifuge (4) wenigstens eine in die Eindickzentrifuge integrierte Lysiereinrichtung zum Aufbrechen der Zellen von in den Abwasserschlämmen enthaltenen Organismen vorgesehen ist.

2. Eindickzentrifuge nach Anspruch 1, dadurch gekennzeichnet, daß sie wenigstens eine Mahleinrichtung und/oder Zerreibeinrichtung aufweist.

3. Eindickzentrifuge nach Anspruch 2, dadurch gekennzeichnet, daß die Eindickzentrifuge (4) eine rotierende Fördereinrichtung (6), insbesondere einen Schneckenförderer, aufweist, welcher den Schlamm zu einem Schlammauslaß (9) befördert, und/oder einen rotierenden Mantel (7) aufweist; und/oder
daß die Eindickzentrifuge (4) eine Düsenzentrifuge oder ein Düsenseparator ist.

4. Eindickzentrifuge nach Anspruch 3, dadurch gekennzeichnet, daß Mantel (7) und Fördereinrichtung (6) mit unterschiedlichen Umfangsgeschwindigkeiten rotieren.

5. Eindickzentrifuge nach einem der Ansprüche 1 bis 4, dadurch gekennzeichnet, daß die Lysiereinrichtung (10; 100; 200; 300; 400; 500; 600; 700) als Friktionsmahlwerk (100; 200) ausgebildet ist.

6. Eindickzentrifuge nach Anspruch 5, dadurch gekennzeichnet, daß das Friktionsmahlwerk (100; 200) wenigstens eine Mahlscheibe (161, 162) zum Zerreiben des Schlammes und der darin enthaltenen Zellen aufweist.

7. Eindickzentrifuge nach Anspruch 6, dadurch gekennzeichnet, daß die Mahlscheibe (161) mit dem Mantel (7) der Eindickzentrifuge (4) rotiert und sich gegen eine weitere feststehende Mahlscheibe (162) bewegt, wobei vorzugsweise der Abstand (170) beider Mahlscheiben (161, 162) einstellbar ist, wobei der Abstand (170) etwa im Bereich von 0,5 bis 5 mm liegt.

8. Eindickzentrifuge nach Anspruch 6 oder 7, dadurch gekennzeichnet. daß wenigstens eine Mahlscheibe an ihrer Mahlfläche (164, 165) Vertiefungen (163), insbesondere Nuten aufweist, welche vorzugsweise einen Anstellwinkel gegenüber der Radialrichtung aufweisen.

9. Eindickzentrifuge nach Anspruch 8, dadurch gekennzeichnet, daß die Nuten von nicht ausgenommen Bereichen der Mahlflächen (164, 165) unterbrochen sind, so daß radiale Nutenreihen ausgebildet sind.

10. Eindickzentrifuge nach Anspruch 9, dadurch gekennzeichnet, daß nebeneinanderliegende Nutenreihen auf Lücke angeordnet sind, so daß Bereiche, an denen eine Nutenreihe unterbrochen ist, die benachbarten Nutenreihen keine Unterbrechung aufweisen.

11. Eindickzentrifuge nach einem der Ansprüche 6 bis 10, dadurch gekennzeichnet, daß die Eindickzentrifuge an ihrem Schlammauslaß ein Wehr (190) aufweist.

12. Eindickzentrifuge nach Anspruch 5, dadurch gekennzeichnet, daß das Friktionsmahlwerk (100; 200) wenigstens einen Mahlkonus (205) zum Zerreiben des Schlammes und der darin enthaltenen Zellen aufweist.

13. Eindickzentrifuge nach Anspruch 12, dadurch gekennzeichnet, daß der Mahl konus (205) einen rotierenden Außenkonus (220) mit wenigstens einer Mahlfläche (261) und einen vorzugsweise feststehenden Innenkonus (230) mit wenigstens einer Gegenfläche (262) aufweist.

14. Eindickzentrifuge nach Anspruch 13, dadurch gekennzeichnet, daß wenigstens der rotierende Außenkonus (220) auf seiner Mahlfläche (261) Vertiefungen (263), insbesondere Nuten, aufweist, wobei die Nutenbreite vorzugsweise in der Größenordnung der Nutentiefe liegt, und wobei die Nuten in Richtung der Mantellinien des Mahlkonus (205) oder in einem Anstellwinkel hierzu angeordnet sind.

15. Eindickzentrifuge nach Anspruch 14, dadurch gekennzeichnet, daß die Nuten von nicht ausgenommenen Bereichen der Mahlflächen (261, 262) unterbrochen sind, so daß Nutenreihen in Richtung der Mantellinien ausgebildet sind.

16. Eindickzentrifuge nach Anspruch 15, dadurch gekennzeichnet, daß nebeneinander liegende Nutenreihen auf Lücke angeordnet sind, so daß Bereiche, an denen eine Nutenreihe unterbrochen ist, die benachbarten Nutenreihen keine Unterbrechung aufweisen.

17. Eindickzentrifuge nach einem der Ansprüche 15 bis 16, dadurch gekennzeichnet, daß Innen- und Außenkonus (230, 220) in einem Abstand (270) voneinander angeordnet sind, wobei der Abstand (270) vorzugsweise mittels Anschlag (275) und Federn während Rotation oder im Stillstand veränderlich und einstellbar ist.

18. Eindickzentrifuge nach einem der Ansprüche 1 bis 4, dadurch gekennzeichnet, daß die Lysiereinrichtung (10; 100; 200; 300; 400; 500; 600; 700) als Profilreibe (305) ausgebildet ist.

19. Eindickzentrifuge nach Anspruch 18, dadurch gekennzeichnet, daß die Profilreibe (305) eine feststehende Außenhülle (330) und eine rotierende Reibfläche (320) aufweist, wobei der Abstand (370) zwischen Außenhülle (330) und Reibfläche (320) einstellbar ist.

20. Eindickzentrifuge nach Anspruch 19, dadurch gekennzeichnet, daß die rotierende Reibfläche (320) Vertiefungen (363), insbesondere wellenförmige Vertiefungen (363) aufweist, wobei die Vertiefungen (363) vorzugsweise einen Anstellwinkel zur Drehrichtung der Profilreibe (305) aufweisen.

21. Eindickzentrifuge nach einem der Ansprüche 18 bis 20, dadurch gekennzeichnet, daß der sich in Richtung des Schlammauslasses (395) erstreckende Teil (326) der Reibfläche (320) eine größere Neigung in Richtung auf die Wand der Außenhülle (330) aufweist, also einen breiteren Spalt (370) aufweist, als der sich in die Gegenrichtung erstreckende Teil (325) der Reibfläche (305).

22. Eindickzentrifuge nach einem der Ansprüche 18 bis 21, dadurch gekennzeichnet, daß die Schlammzuführung zur Profilreibe (305) vorzugsweise zentrisch vorgesehen ist.

23. Eindickzentrifuge nach einem der Ansprüche 18 bis 21, dadurch gekennzeichnet, daß der Abstand (370) zwischen Reibfläche (320) und Außenhülle (330) minimal ca. 2 mm bis maximal ca. 10 mm beträgt.

24. Eindickzentrifuge nach einem der Ansprüche 1 bis 4, dadurch gekennzeichnet, daß die Lysiereinrichtung (10; 100; 200; 300; 400; 500; 600; 700) als Walzenquetsche (405) ausgebildet ist.

25. Eindickzentrifuge nach Anspruch 24, dadurch gekennzeichnet, daß die Walzenquetsche (405) wenigstens einen Walzensatz (441, 442) aufweist, der auf der Innenwand einer vorzugsweise feststehenden Außenhülle (430) in Innenumfangsrichtung der Außenhülle (430) rollt.

26. Eindickzentrifuge nach Anspruch 25, dadurch gekennzeichnet, daß der Walzensatz (441, 442) wenigstens zehn Walzen aufweist.

27. Eindickzentrifuge nach einem der Ansprüche 1 bis 4, dadurch gekennzeichnet, daß die Lysiereinrichtung (10; 100; 200; 300; 400; 500; 600; 700) als Passiertrommel (505) ausgebildet ist.

28. Eindickzentrifuge nach Anspruch 27, dadurch gekennzeichnet, daß die Passiertrommel (505) mehrere Passierelemente aufweist, welche in einer feststehenden Außenhülle mit einem vorzugsweise einstellbaren Abstand (570) zur Innenwand der Außenhülle (530) rotieren.

29. Eindickzentrifuge nach einem der Ansprüche 1 bis 4, dadurch gekennzeichnet, daß die Lysiereinrichtung (10; 100; 200; 300; 400; 500; 600; 700) als Schneidwerk (605) ausgebildet ist.

30. Eindickzentrifuge nach Anspruch 29, dadurch gekennzeichnet, daß das Schneidwerk (605) rotierende Schneidelementreihen (641), insbesondere Messerreihen (641), feststehende Schneidelemente, insbesondere Messerreihen (641) sowie ein Wehr (690) am Schlammauslaß (695) aufweist, wobei die Schneidelementereihen (641) ineinander eingreifen ohne sich zu berühren.

31. Eindickzentrifuge nach Anspruch 30, dadurch gekennzeichnet, daß der Abstand (670), die Höhe und die Umfangslänge der Messer der feststehenden und der rotierenden Messerreihen (641) einstellbar ist.

32. Eindickzentrifuge nach einem der Ansprüche 29 bis 31, dadurch gekennzeichnet, daß die Messer (661, 662) mit einer Geschwindigkeit von ca. 50 bis 100 m/s, vorzugsweise ca. 80 m/s rotieren.

33. Eindickzentrifuge nach einem der Ansprüche 29 bis 32, dadurch gekennzeichnet, daß eine Messerreihe (641) eine Mehrzahl von Messern (661, 662) aufweist, wobei nebeneinander liegende Messerreihen auf Lücke angeordnet sind, so daß Bereiche, an denen eine Messerreihe (661, 662) unterbrochen ist, die benachbarten Messerreihen (641) keine Unterbrechung aufweisen.

34. Eindickzentrifuge nach einem der Ansprüche 29 bis 33, dadurch gekennzeichnet, daß die Messerenden (663) einen Anstellwinkel zur Rotationsrichtung des Schneidwerks (605) aufweisen.

35. Eindickzentrifuge nach einem der Ansprüche 29 bis 34, dadurch gekennzeichnet, daß die Höhe des Wehrs (690) am Schlammauslaß (695) einstellbar ist.

36. Eindickzentrifuge nach einem der Ansprüche 1 bis 4, dadurch gekennzeichnet, daß die Lysiereinrichtung (10; 100; 200; 300; 400; 500; 600; 700) als Stiftmahlwerk (705) ausgebildet ist.

37. Eindickzentrifuge nach Anspruch 36, dadurch gekennzeichnet, daß das Stiftmahlwerk rotierende Stiftreihen (761; 741), feststehende Stiftreihen (762; 741) sowie ein Wehr (790) am Schlammauslaß (795) aufweist, wobei die rotierenden und die feststehenden Stiftreihen (741) ineinander eingreifen ohne sich zu berühren.

38. Eindickzentrifuge nach Anspruch 37, dadurch gekennzeichnet, daß der Abstand (770) zwischen den feststehenden und den rotierenden Stiftreihen (761, 762)einstellbar ist.

39. Eindickzentrifuge nach einem der Ansprüche 36 bis 38, dadurch gekennzeichnet, daß die Stifte (761) mit einer Geschwindigkeit von ca. 50 bis 100 m/s, vorzugsweise ca. 80 m/s rotieren.

40. Eindickzentrifuge nach einem der Ansprüche 36 bis 39, dadurch gekennzeichnet, daß die Stifte (761, 762) derart angeordnet sind, daß nebeneinander liegende Stiftreihen (741) auf Lücke angeordnet sind, so daß Bereiche, an denen keine Stifte in einer Stiftreihe (741) vorhanden sind, die benachbarten Stiftreihen (741) Stifte (761, 762) aufweisen.

41. Eindickzentrifuge nach einem der Ansprüche 36 bis 40, dadurch gekennzeichnet, daß die Stifte (761, 762) einen Winkel zur Drehachse einschließen.

42. Eindickzentrifuge nach einem der Ansprüche 36 bis 41, dadurch gekennzeichnet, daß die Höhe des Wehrs (790) am Schlammauslaß (795) einstellbar ist.

43. Verfahren zur Verminderung der Faulschlammenge bzw. Restschlammenge in Kläranlagen, welches die folgenden Schritte umfaßt:
Absetzenlassen von Abwasser in wenigstens einem Absetzbecken (802) und Fördern des sedimentierten Überschußschlammes (807) zu wenigstens einem anaeroben Reaktor (812) mittels wenigstens einer Fördereinrichtung sowie Weiterleiten des Abwassers in eine aerobe Belebungseinrichtung (803);
aerobes Umsetzen des abgesetzten Abwassers in wenigstens einer aeroben Belebungseinrichtung (803);
Eindicken des Überschußschlammes (807) aus einem Absetzbecken (804) in wenigstens einer Eindickzentrifuge (4) als Eindickungseinrichtung;
Umsetzen des Überschußschlammes (807) in wenigstens einem anaeroben Reaktor (812);
**dadurch gekennzeichnet, daß**
in der Eindickzentrifuge (4) eine Menge von ca. 0,5 bis 50 % der Menge an ursprünglich in dem Überschußschlamm (807) enthaltenen Mikroorganismenzellen bei der Eindickung vor dem anaeroben Reaktor (812) lysiert wird.

44. Verfahren nach Anspruch 43, dadurch gekennzeichnet, daß in dem anaeroben Reaktor (812) ein brennbares Gas, insbesondere Methan, erzeugt wird.

45. Verfahren nach einem der Ansprüche 43 bis 44, dadurch gekennzeichnet, daß das in dem Verfahren erzeugte brennbare Gas zur Stromerzeugung verwendet wird, wobei vorzugsweise der erzeugte Strom zum Betreiben der Kläranlage genutzt wird.

46. Verfahren nach einem der Ansprüche 43 bis 45, dadurch gekennzeichnet, daß das durch das Verfahren die Produktion von brennbarem Gas beschleunigt wird und das Volumen desselben insbesondere um 10 bis 50% vergrößert wird.

## Claims

1. Thickening ccntrifuge for thickening surplus sludge (807),
characterized in that
in the thickening centrifuge (4), at least one lysis device, integrated in the thickening centrifuge, is provided for breaking up the cells of organism contained in the waste-water sludges.

2. Thickening centrifuge according to Claim 1, characterized in that it comprises at least one grinding device and/or crushing device.

3. Thickening centrifuge according to Claim 2, characterized in that the thickening centrifuge (4) comprises a rotating conveyor device (6), particularly a screw conveyor, which supplies the sludge to a sludge outlet (9), and/or comprises a rotating shell (7), and/or
in that the thickening centrifuge (4) is a nozzle centrifuge or a nozzle separator.

4. Thickening centrifuge according to Claim 3, characterized in that the shell (7) and the conveyor device (6) rotate with different circumferential velocities.

5. Thickening centrifuge according to any one of Claims 1 to 4, characterized in that the lysis device (10;, 100; 200; 300; 400; 500; 600; 700) is formed as a friction grinder (100;200).

6. Thickening centrifuge according to Claim 5, characterized in that the friction grinder (100; 200) comprises at least one grinding disk (161, 162) for crushing the sludge and the cells contained therein.

7. Thickening centrifuge according to Claim 6, characterized in that the grinding disk (161) rotates with the shell (7) of the thickening centrifuge (4) and moves against a further stationary grinding disc (162), the spacing (170) of the two grinding disks (161, 162) preferably being adjustable, the spacing (170) being approximately in the range of from 0.5 to 5 mm.

8. Thickening centrifuge according to Claim 6 or Claim 7, characterized in that at least one grinding disk comprises, on its grinding surface (164, 165), recesses (163), particularly grooves, which are preferably inclined at an angle of attack to the radial direction.

9. Thickening centrifuge according to Claim 8, characterized in that the grooves are interrupted by non-recessed regions of the grinding surfaces (164, 165), so that radial rows of grooves are formed.

10. Thickening centrifuge according to Claim 9, characterized in that rows of grooves disposed side by side are staggered so that, in regions in which one row of grooves is interrupted, the adjacent rows of grooves have no interruption.

11. Thickening centrifuge according to any one of Claims 6 to 10, characterized in that the thickening centrifuge comprises a gate (190) on its sludge outlet.

12. Thickening centrifuge according to Claim 5, characterized in that he friction grinder (100; 200) comprises at least one milling cone (205) for crushing the sludge and the cells contained therein.

13. Thickening centrifuge according to Claim 12, characterized in that the milling cone (205) comprises a rotating outer cone (220) with at least one milling surface (261) and a preferably stationary inner cone (230) with at least one opposed surface (262).

14. Thickening centrifuge according to Claim 13, characterized in that at least the rotating outer cone (220) comprises, on its milling surface (161), recesses (263), particularly grooves, the groove width preferably lying in the order of magnitude of the groove depth and the grooves being arranged in the direction of the generatrices of the milling cone (205) or being inclined at an angle of attack thereto.

15. Thickening centrifuge according to Claim 14, characterized in that the grooves are interrupted by non-recessed regions of the milling surfaces (261, 262) so that rows of grooves are formed in the direction of the generatrices.

16. Thickening centrifuge according to Claim 15, characterized in that rows of grooves disposed side by side are staggered so that, in regions in which one row of grooves is interrupted, the adjacent rows of grooves have no interruption.

17. Thickening centrifuge according to any one of Claims 15 to 16, characterized in that the inner and outer cones (230, 220) are arranged with a spacing (270) relative to one another, the spacing (270) preferably being variable and adjustable, during rotation or in the stationary condition, by means of a stop (275) and springs.

18. Thickening centrifuge according to any one of Claims 1 to 4, characterized in that the lysis device (10; 100; 200; 300; 400; 500; 600; 700) is formed as a shaped rasp (305).

19. Thickening centrifuge according to Claim 18, characterized in that the shaped rasp (305) comprises a stationary outer case (330) and a rotating rasp surface (320), the spacing (370) between outer case (330) and rasp surface (320) being adjustable.

20. Thickening centrifuge according to Claim 19, characterized in that the rotating rasp surface (320) comprises recesses (363), particularly undulating recesses (363), the recesses (363) preferably being inclined at an angle of attack to the direction of rotation of the shaped rasp (305).

21. Thickening centrifuge according to any one of Claims 18 to 20, characterized in that the portion (326) of the rasp surface (320) extending in the dircction of the sludge outlet (395) has a greater inclination to the wall of the outer case (330), and hence has a wider gap (370) than the portion (325) of the rasp surface (305) extending in the opposite direction.

22. Thickening centrifuge according to any one of Claims 18 to 21, characterized in that the sludge supply to the shaped rasp (305) is preferably arranged centrally.

23. Thickening centrifuge according to any one of Claims 18 to 21, characterized in that the spacing (370) between *rasp* surface (320) and outer case (33) is from a minimum of approximately 2 mm up to a maximum of approximately 10 mm.

24. Thickening centrifuge according to any one of Claims 1 to 4, characterized in that the lysis device (10; 100; 200; 300; 400; 500; 600; 700) is formed as a roller crusher (405).

25. Thickening centrifuge according to Claim 24, characterized in that the roller crusher (405) comprises at least one set of rollers (441, 442) which rolls on the inner wall of a preferably stationary outer case (430) in the direction of the inner circumference of the outer case (430).

26. Thickening centrifuge according to Claim 25, characterized in that the set of rollers (441, 442) comprises at least ten rollers.

27. Thickening centrifuge according to any one of Claims 1 to 4, characterized in that the lysis device (10; 100, 200; 300; 400; 500; 600; 700) is formed as a passing drum (505).

28. Thickening centrifuge according to Claim 27, characterized in that the passing drum (505) comprises a plurality of passing elements which rotate in a stationary outer case preferably with an adjustable spacing (570) from the inner wall of the outer case (530).

29. Thickening centrifuge according to any one of Claims 1 to 4, characterized in that the lysis device (10; 100, 200; 300; 400; 500; 600; 700) is formed as a cutting mechanism (605).

30. Thickening centrifuge according to Claim 29, characterized in that the cutting mechanism (605) comprises rotating rows of cutting elements (641), particularly rows of cutters (641), stationary cutting elements, particularly rows of cutters (641), and a gate (690) at the sludge outlet (695), the rows of cutting elements (641) meshing with one another without contact.

31. Thickening centrifuge according to Claim 30, characterized in that the spacing (670), the height, and the circumferential length of the cutters of the stationary and of the rotating rows of cutters (641) is adjustable.

32. Thickening centrifuge according to anyone of Claims 29 to 31, characterized in that the cutters (661, 662) rotate at a speed of approximately 50 to 100 m/s, preferably approximately 80 m/s.

33. Thickening centrifuge according to any one of Claims 29 to 32, characterized in that a raw of cutters (641) comprises a plurality of cutters (661, 662), rows of cutters disposed side by side being staggered so that, in regions in which one row of cutters (661, 662) is interrupted the adjacent rows of cutters (641) have no interruption.

34. Thickening centrifuge according to any one of Claims 29 to 33, characterized in that the ends of the cutters (663) are inclined at an angle of attack relative to the direction of rotation of the cutting mechanism (605).

35. Thickening centrifuge according to any one of Claims 29 to 34, characterized in that the height of the gate (690) at Lhe sludge outlet (695) is adjustable.

36. Thickening centrifuge according to any one of Claims 1 to 4, characterized in that the lysis device (10; 100, 200, 300; 400; 500; 600; 700) is formed as a pin grinder (705),

37. Thickening centrifuge according to Claim 36, characterized in that the pin grinder comprises rotating rows of pins (761; 741), stationary rows of pins (762; 741), and a gate (790) at the sludge outlet (795), the rotating and stationary rows of pins (741) meshing with one another without contact.

38. Thickening centrifuge according to Claim 37, characterized in that the spacing (770) between the stationary and the rotating ruws of pins (761, 762) is adjustable.

39. Thickening centrifuge according to any one of Claims 36 to 38, characterized in that the pins (761) rotate at a speed of approximately 50 to 100 m/s, preferably approximately 80 m/s.

40. Thickening centrifuge according to any one of Claims 36 to 39, charactcrized in that the pins (761, 762) are arranged in a manner such that rows of pins (741) disposed side by side are staggered so that, in regions in which there are no pins in one row of pins (741), the adjacent rows of pins (741) have pins (761, 762).

41. Thickening centrifuge according to any one of Claims 36 to 40, characterized in that the pins (761, 762) are inclined at an angle relative to the axis of rotation.

42. Thickening centrifuge according to any one of Claims 36 to 41, characterized in that the height of the gate (790) at the sludge outlet (795) is adjustable.

43. Process for reducing digested sludges and/or residual sludges in sewage-treatment works, which comprises the following steps:
leaving waste water to settle in at least one settlement tank (802) and conveying the sedimented excess sludge (807) to at least one anaerobic reactor (812) by means of at least one conveyor device and passing the waste water to an aerobic activation device (803);
aerobic conversion of the waste water drawn off in at least one aerobic activation device (803);
thickening of the surplus sludge (807) from a settlement tank (804) in at least one thickening centrifuge (4) as thickening device;
transferring the surplus sludge (807) to at least one anaerobic reactor (812) ;
characterized in that
a quantity of from approximately 0.5 to 50% of the quantity of micro-organism cells originally contained in the surplus sludge (807) is lysed in the thickening centrifuge (4) during the thickening, upstream of the anaerobic reactor (812).

44. Process according to Claim 43, characterized in that a combustible gas, particularly methane, is produced in the anaerobic reactor (812).

45. Process according to any one of Claims 43 to 44, characterized in that combustible gas produced in the process is used for power generation, the power produced preferably being utilized for operating the sewage-treatment plant.

46. Process according to any one of Claims 43 to 45, characterized in that the production of combustible gas is promoted by the process and the volume thereof is increased, particularly by 10 to 50%.

## Revendications

1. Centrifugeuse d'épaississement pour épaissir une boue résiduelle (807),
caractérisée en ce que
au moins un dispositif provoquant un effet lysogène, qui est intégré à la centrifugeuse d'épaississement, est prévu dans la centrifugeuse d'épaississement (4) pour dissocier les cellules d'organismes que contiennent les boues d'eaux usées.

2. Centrifugeuse d'épaississement selon la revendication 1, caractérisée en ce qu'elle comprend au moins un dispositif de broyage et/ou un dispositif de trituration.

3. Centrifugeuse d'épaississement selon la revendication 2, caractérisée en ce que la centrifugeuse d'épaississement (4) comporte un dispositif rotatif de transport (6), en particulier un transporteur à vis sans fin, qui transporte la boue vers une sortie de boue et/ou comprend une enveloppe rotative (7) et/ou
en ce que la centrifugeuse d'épaississement (4) est une centrifugeuse à buses ou un séparateur à buses.

4. Centrifugeuse d'épaississement selon la revendication 3, caractérisée en ce que l'enveloppe (7) et le dispositif de transport (6) tournent à des vitesses circonférentielles différentes.

5. Centrifugeuse d'épaississement selon l'une des revendications 1 à 4, caractérisée en ce que le dispositif provoquant un effet lysogène (10 ; 100 ; 200 ; 300 ; 400 ; 500 ; 600 ; 700) est conformé en broyeur à friction (100 ; 200).

6. Centrifugeuse d'épaississement selon la revendication 5, caractérisée en ce que le broyeur à friction (100 ; 200) comporte au moins un disque de broyage (161, 162) pour la trituration de la boue et des cellules que celle-ci contient.

7. Centrifugeuse d'épaississement selon la revendication 6, caractérisée en ce que le disque de broyage (161) tourne avec l'enveloppe (7) de la centrifugeuse d'épaississement (4) et se déplace contre un autre disque fixe de broyage (162), la distance (170) séparant les deux disques de broyage (161, 162) étant de préférence réglable, la distance (170) étant comprise dans une plage approximative de 0,5 à 5 mm.

8. Centrifugeuse d'épaississement selon la revendication 6 ou 7, caractérisée en ce qu'au moins un disque de broyage comporte sur sa surface de broyage (164, 165) des cavités (163), en particulier des rainures, qui ont de préférence un angle d'obliquité sur la direction radiale.

9. Centrifugeuse d'épaississement selon la revendication 8, caractérisée en ce que les rainures sont interrompues par des zones non évidées des surfaces de broyage (164, 165), de manière que des rangées radiales de rainures soient réalisées.

10. Centrifugeuse d'épaississement selon la revendication 9, caractérisée en ce que les rangées juxtaposées de rainures sont disposées en quinconce de manière que dans les zones dans lesquelles une rangée de rainures est interrompue, les rangées voisines de rainures ne comportent pas une interruption.

11. Centrifugeuse d'épaississement selon l'une des revendications 6 à 10, caractérisée en ce que la centrifugeuse d'épaississement comporte un barrage (190) à sa sortie de boue.

12. Centrifugeuse d'épaississement selon la revendication 5, caractérisée en ce que le broyeur à friction (100 ; 200) comporte au moins un cône de broyage (205) pour la trituration de la boue et des cellules que celle-ci contient.

13. Centrifugeuse d'épaississement selon la revendication 12, caractérisée en ce que le cône de broyaqe (205) comporte un cône rotatif extérieur (220) présentant au moins une surface de broyage (261), ainsi qu'un cône interne de préférence fixe (230) ayant au moins une surface complémentaire (262).

14. Centrifugeuse d'épaississement selon la revendication 13, caractérisée en ce qu'au moins le cône extérieur rotatif (220) comporte sur sa surface de broyage (261) des cavités (263), en particulier des rainures, la largeur des rainures étant de préférence de l'ordre de grandeur de la profondeur des rainures et les rainures étant disposées dans la direction des génératrices du cône de broyage (205) ou suivant un angle d'obliquité par rapport à celles-ci.

15. Centrifugeuse d'épaississement selon la revendication 14, caractérisée en ce que les rainures sont interrompues par des zones non évidées des surfaces de broyage (261, 262), de manière que les rangées de rainures soient réalisées dans la direction des génératrices.

16. Centrifugeuse d'épaississement selon la revendication 15, caractérisée en ce que les rangées juxtaposées de rainures sont disposées en quinconce de manière que dans les zones dans lesquelles une rangée de rainures est interrompue, les rangées voisines de rainures ne comportent aucune interruption.

17. Centrifugeuse d'épaississement selon l'une des revendications 15 et 16, caractérisée en ce que les cônes intérieur et extérieur (230, 220) sont disposés à distance (270) l'un de l'autre, la distance (270) étant modifiable et réglable de préférence au moyen d'une butée (275) et de ressorts pendant la rotation ou à l'arrêt.

18. Centrifugeuse d'épaississement selon l'une des revendications 1 à 4, caractérisée en ce que le dispositif provoquant un effet lysogène (10 ; 100 ; 200 ; 300 ; 400 ; 500 ; 600 ; 700) est conformé en râpe profilée (305).

19. Centrifugeuse d'épaississement selon la revendication 18, caractérisée en ce que la râpe profilée (305) comporte une gaine extérieure fixe (330) et une surface rotative de râpe (320), la distance (370) séparant la gaine extérieure (330) et la surface de râpe (320) étant réglable.

20. Centrifugeuse d'épaississement selon la revendication 19, caractérisée en ce que la surface rotative de râpe (320) comporte des cavités (363), en particulier des cavités ondulées (363), les cavités (363) présentant de préférence un angle d'obliquité sur la direction de rotation de la râpe profilée (305).

21. Centrifugeuse d'épaississement selon l'une des revendications 18 à 20, caractérisée en ce que la partie (326) de la surface de râpe (320) qui est orientée vers la sortie de la boue (395) présente une inclinaison vers la paroi de la gaine extérieure (330) supérieure à , donc un interstice plus large (370) que, la partie (325) de la surface de râpe (305) qui est orientée en sens inverse.

22. Centrifugeuse d'épaississement selon l'une des revendications 18 à 21, caractérisée en ce que l'arrivée de boue sur la râpe profilée (305) est de préférence prévue centralement.

23. Centrifugeuse d'épaississement selon l'une des revendications 18 à 21, caractérisée en ce que la distance (370) comprise entre la surface de râpe (320) et la gaine extérieure (330) est au minimum d'environ 2 mm à un maximum d'environ 10 mm.

24. Centrifugeuse d'épaississement selon l'une des revendications 1 à 4, caractérisée en ce que le dispositif produisant un effet lysogène (10 ; 100 ; 200 ; 300 ; 400 ; 500 ; 600 ; 700) est conformé en presse à cylindres (405).

25. Centrifugeuse d'épaississement selon la revendication 24, caractérisée en ce que la presse à cylindres (405) comprend de préférence un groupe de cylindres (441, 442) qui roule sur la paroi intérieure d'une gaine extérieure de préférence fixe (430) dans la direction de la circonférence intérieure de la gaine extérieure (430).

26. Centrifugeuse d'épaississement selon la revendication 25, caractérisée en ce que le groupe de cylindres (441, 442) comprend au moins dix cylindres.

27. Centrifugeuse d'épaississement selon l'une des revendications 1 à 4, caractérisée en ce que le dispositif provoquant un effet lysogène (10 ; 100 ; 200 ; 300 ; 400 ; 500 ; 600 ; 700) est conformé en tambour hachoir (505).

28. Centrifugeuse d'épaississement selon la revendication 27, caractérisée en ce que le tambour hachoir (505) comporte plusieurs éléments hachoirs qui tournent dans une gaine extérieure fixe à une distance (570) de préférence réglable à la paroi intérieure de la gaine extérieure (530).

29. Centrifugeuse d'épaississement selon l'une des revendications 1 à 4, caractérisée en ce que le dispositif provoquant un effet lysogène (10 ; 100 ; 200 ; 300 ; 400 ; 500 ; 600 ; 700) est conformé en mécanisme de coupe (605).

30. Centrifugeuse d'épaississement selon la revendication 29, caractérisée en ce que le mécanisme de coupe (605) comporte des rangées rotatives d'éléments de coupe (641), en particulier des rangées de lames (641), des éléments fixes de coupe, en particulier des rangées de lames (641), ainsi qu'un barrage (690) à la sortie (695) de la boue, les rangées d'éléments de coupe (641) s'imbriquant sans se toucher.

31. Centrifugeuse d'épaississement selon la revendication 30, caractérisée en ce que la distance (670), la hauteur et la longueur circonférentielle des lames des rangées fixes et des rangées rotatives de lames (641) sont réglables.

32. Centrifugeuse d'épaississement selon l'une des revendications 29 à 31, caractérisée en ce que les lames (661, 662) tournent à une vitesse d'environ 50 à 100 m/s, de préférence d'environ 80 m/s.

33. Centrifugeuse d'épaississement selon l'une des revendications 29 à 32, caractérisée en ce qu'une rangée de lames (641) comprend plusieurs lames (661, 662), les rangées juxtaposées de lames étant disposées en quinconce de manière que dans les zones dans lesquelles une rangée de lames (661, 662) est interrompue, les rangées voisines de lames (641) ne comportent aucune interruption.

34. Centrifugeuse d'épaississement selon l'une des revendications 29 à 33, caractérisée en ce que les extrémités (663) des lames présentent un angle d'obliquité sur la direction de rotation du mécanisme de coupe (605).

35. Centrifugeuse d'épaississement selon l'une des revendications 29 à 34, caractérisée en ce que la hauteur du barrage (690) à la sortie (695) de la boue est réglable.

36. Centrifugeuse d'épaississement selon l'une des revendications 1 à 4, caractérisée en ce que le dispositif provoquant un effet lysogène (10 ; 100 ; 200 ; 300 ; 400 ; 500 ; 600 ; 700) est conformé en broyeur à chevilles (705),

37. Centrifugeuse d'épaississement selon la revendication 36, caractérisée en ce que le broyeur à chevilles comprend des rangées rotatives de chevilles (761 ; 741), des rangées fixes de chevilles (762 ; 741) ainsi qu'un barrage (790) à la sortie (795) de la boue, les rangées rotatives et les rangées fixes de chevilles (741) s'imbriquant sans se toucher.

38. Centrifugeuse d'épaississement selon la revendication 37, caractérisée en ce que la distance (770) séparant les rangées fixes et les rangées rotatives de chevilles (761, 762) est réglable.

39. Centrifugeuse d'épaississement selon l'une des revendications 36 à 38, caractérisée en ce que les chevilles (761) tournent à une vitesse d'environ 50 à 100 m/s, de préférence d'environ 80 m/s.

40. Centrifugeuse d'épaississement selon l'une des revendications 36 à 39, caractérisée en ce que les chevilles (761, 762) sont disposées de manière que les rangées juxtaposées de chevilles (741) soient disposées en quinconce de façon que dans les zones dans lesquelles aucune cheville n'est présente dans une rangée de chevilles (741), les rangées voisines de chevilles (741) comportent des chevilles (761, 762).

41. Centrifugeuse d'épaississement selon l'une des revendications 36 à 40, caractérisée en ce que les chevilles (761, 762) inscrivent un angle avec l'axe de rotation.

42. Centrifugeuse d'épaississement selon l'une des revendications 36 à 41, caractérisée en ce que la hauteur du barrage (790) à la sortie (795) de la boue est réglable.

43. Procédé de réduction de la quantité de boue putréfiée ou de la quantité de boue résiduelle dans des installations d'épuration, qui comprend les étapes suivantes :
décantation des eaux usées dans au moins un bassin de décantation (802) et transport de la boue résiduelle sédimentée (807) vers au moins un réacteur anaérobie (812) au moyen d'au moins un dispositif de transport ainsi que transfert des eaux usées dans un dispositif aérobie d'activation (803) ;
décomposition aérobie de l'eau usée décantée dans au moins un dispositif aérobie d'activation (803) ;
épaississement de la boue résiduelle (807) sortant d'un bassin de décantation (804) dans au moins une centrifugeuse d'épaississement (4) servant de dispositif d'épaississement ;
décomposition de la boue résiduelle (807) dans au moins un réacteur anaérobie (812) ;
caractérisé en ce qu'une quantité d'environ 0,5 à 50% de la quantité des cellules de micro-organismes que contient initialement la boue résiduelle (807) subit un effet lysogène dans la centrifugeuse d'épaississement (4) lors de l'épaississement effectué en amont du réacteur anaérobie (812).

44. Procédé selon la revendication 43, caractérisé en ce qu'un gaz combustible, en particulier du méthane, est généré dans le réacteur anaérobie (812).

45. Procédé selon l'une des revendications 43 et 44, caractérisé en ce que le gaz combustible généré par le procédé est utilisé pour la génération de courant, le courant généré étant de préférence utilisé pour faire fonctionner l'installation d'épuration.

46. Procédé selon l'une des revendications 43 à 45, caractérisé en ce que la production de gaz combustible par le procédé est accélérée et le volume de ces gaz est accru en particulier de 10 à 50%.
